# EUROPÄISCHE PATENTSCHRIFT

(11) **EP 3 705 178 B1**
(45) Veröffentlichungstag und Bekanntmachung des Hinweises auf die Patenterteilung: **30.08.2023**
(21) Anmeldenummer: 19020106.1
(22) Anmeldetag: 06.03.2019
(51) Int. Cl.: B01J 12/00, C10L 3/12, C10G 3/00, C07C 1/20, B01J 19/30, B01J 8/04

(54) **VERFAHREN UND ANLAGE ZUR HERSTELLUNG VON OLEFINEN AUS OXYGENATEN**
METHOD AND SYSTEM FOR THE PRODUCTION OF OLEFINS FROM OXYGENATES
PROCÉDÉ ET INSTALLATION DE PRODUCTION DES OLÉFINES À PARTIR DES PRODUITS OXYGÉNÉS

(43) Veröffentlichungstag der Anmeldung: 09.09.2020
(73) Patentinhaber: L'AIR LIQUIDE, SOCIÉTÉ ANONYME POUR L'ÉTUDE ET L'EXPLOITATION DES PROCÉDÉS GEORGES CLAUDE, 75007 Paris (FR)
(72) Erfinder: JANKO, Lutz, 64390 Erzhausen (DE); GORNY, Martin, 65760 Eschborn (DE); HAAG, Stéphane, 60598 Frankfurt am Main (DE); AHLERS, Bernd, 63128 Dietzenbach (DE)
(74) Vertreter: Dropsch, Holger

(56) Entgegenhaltungen:
- EP-A1- 2 032 245
- EP-A1- 2 760 809

## Beschreibung

### Gebiet der Erfindung

Die Erfindung betrifft ein Verfahren zur Herstellung eines Olefine enthaltenden Kohlenwasserstoffproduktes, umfassend insbesondere Ethylen und Propylen, durch Umsetzung eines Oxygenate enthaltenden Eduktgemischs, das in mehrere Eduktgemisch-Teilströme aufgeteilt wird, in einem mehrstufigen Oxygenat-zu-Olefin-(OTO)-Synthesereaktor mit mehreren, hintereinander geschalteten Reaktionssektionen, die jeweils Katalysatorzonen mit festen, für die OTO-Synthese aktiven und selektiven Katalysatoren enthalten, wobei stromaufwärts jeder Katalysatorzone eine Zugabevorrichtung für einen Eduktgemisch-Teilstrom angeordnet ist. In jede dieser Reaktionssektionen wird ein Eduktgemisch-Teilstrom eingeleitet und dort unter Oxygenatumsetzungsbedingungen zu Olefinen und weiteren Kohlenwasserstoffen umgesetzt, wobei alle Reaktionssektionen außer der ersten zusätzlich mit dem Produktstrom der jeweils stromaufwärts angeordneten Reaktionssektion beaufschlagt werden. Zusätzlich werden mindestens ein Wasserdampfstrom in mindestens eine Reaktionssektion und mindestens ein Kohlenwasserstoffe enthaltender Rückführstrom in mindestens eine Reaktionssektion eingeleitet. Das OTO-Synthesereaktorprodukt wird in einer mehrstufigen Aufarbeitungsvorrichtung fraktioniert, wobei mehrere, Kohlenwasserstoffe enthaltende Kohlenwasserstoff-Produktfraktionen erhalten werden, von denen mindestens eine als Rückführstrom zum OTO-Synthesereaktor zurückgeführt wird.

Die Erfindung betrifft ferner eine Anlage zur Durchführung eines solchen Verfahrens.

### Stand der Technik

Kurzkettige Olefine, insbesondere Propylen (Propen), gehören zu den wichtigsten Grundstoffen der chemischen Industrie. Dies liegt darin begründet, dass ausgehend von diesen ungesättigten Verbindungen mit einer kurzen Kettenlänge Moleküle mit langkettigem Kohlenstoffgerüst und zusätzlichen Funktionalisierungen aufgebaut werden können.

Als Quelle für kurzkettige Olefine diente in der Vergangenheit vor allem das Steam-Cracking, d.h. die thermische Spaltung bei der Erdölverarbeitung. In den vergangenen Jahren wurden jedoch weitere Prozesse zur Herstellung von kurzkettigen Olefinen entwickelt. Dies ist zum einen durch steigende Nachfrage bedingt, die durch die vorhandenen Quellen nicht mehr gedeckt werden kann; zum anderen fordert die zunehmende Verknappung von fossilen Rohstoffen die Verwendung anderer Ausgangsstoffe.

Die sog. MTP- (Methanol-to-Propylen) oder auch MTO- (Methanol-to-Olefin) Verfahren zur Herstellung von Propylen und anderen kurzkettigen Olefinen gehen von Methanol als Ausgangsstoff aus. Verallgemeinernd spricht man in diesem Zusammenhang auch von Oxygenat-zu-Olefin-(OTO)-Verfahren, da sauerstoffhaltige organische Komponenten wie Methanol oder Dimethylether (DME) auch als Oxygenate bezeichnet werden. In diesen heterogen katalysierten Verfahren wird demnach beispielsweise aus Methanol zunächst teilweise das Zwischenprodukt Dimethylether und nachfolgend aus einer Mischung von Methanol und Dimethylether eine Mischung aus Ethylen und Propylen sowie Kohlenwasserstoffen mit höherer Molmasse, darunter vor allem auch Olefine, gebildet. Zudem findet sich im Produktstrom Wasser, welches zum einen aus dem Prozessdampf stammt, der optional dem OTO-Reaktor zur Reaktionsmodulierung zugeführt wird und dem im OTO-Reaktor erzeugten Reaktionswasser.

Im dem aus dem Stand der Technik bekannten MTP-Verfahren ist Reinmethanol Ausgangsstoff für die Umsetzung, das zunächst destillativ aus Rohmethanol gewonnen wird. Da die Kohlenwasserstoffsynthese ausgehend von Methanol im OTO-Reaktor stark exotherm verläuft, wird zunächst in einem dem OTO-Reaktor vorgeschalteten Veretherungsreaktor, dem sogenannten DME-Reaktor, Reinmethanol nach Verdampfen zugeführt und dort nach optionaler Zugabe von Wasserdampf als Verdünnungsmedium im geraden Durchgang mittels heterogen-katalysierter Dehydratisierung zu Dimethylether (DME) und Wasser umgesetzt. Das so entstehende Produktgemisch enthält neben DME noch nicht umgesetztes Methanol und Wasser, es wird nach Ausleiten aus dem DME-Reaktor üblicherweise gekühlt und teilkondensiert, wobei eine DME-reiche Gasphase und eine wasser- und methanolreiche Flüssigphase als erhalten werden, die beide als Eduktgemisch für die nachfolgende OTO-Reaktion eingesetzt werden.

Die nachfolgende Umsetzung des vorreagierten, DME und Methanol als Oxygenate enthaltenden Einsatzgemischs in einem mehrstufigen OTO-Reaktor wird beispielsweise in der europäischen Patentschrift EP 2032245 B1 gelehrt. Der OTO-Reaktor umfasst dabei mehrere innerhalb eines geschlossenen, stehenden Behälters angeordnete, von dem Oxygenate enthaltenden Einsatzgemisch von oben nach unten durchströmte Reaktionssektionen, jeweils bestehend aus einem Tragboden mit einer darauf befindlichen, aus einer Schüttung aus körnigem Molekularsieb-Katalysator beispielsweise des Strukturtyps ZSM-5 gebildeten Katalysatorzone. In einem jeweils von zwei benachbarten Reaktionssektionen nach oben und unten begrenzten Zwischenraum ist ein Zerstäubersystem in Form einer Schar von Düsenrohren mit Zweistoffdüsen angeordnet, das zum gleichmäßigen Versprühen der aus dem DME-Reaktor erhaltenen, wasser- und methanolreiche Flüssigphase mittels der aus dem DME-Reaktor erhaltenen, DME-reichen Gasphase als Treibmittel dient. Neben der hierdurch bewirkten, feinen Verteilung des Eduktgemischs besteht ein weiterer Vorteil darin, dass die zur Verdampfung der feinen Flüssigkeitströpfchen benötigte Verdampfungsenthalpie den Reaktionssektionen und insbesondere den Katalysatorzonen entzogen wird und somit für deren Kühlung sorgt, wodurch die starke Wärmetönung der OTO-Reaktion gut kontrolliert werden kann. Diese würde ansonsten dazu führen, dass die Reaktionstemperatur und somit die Verfahrensbedingungen im OTO-Reaktor lokal stark variieren und somit weder innerhalb einer Reaktionssektion noch über den gesamten OTO-Reaktor überall eine optimale Prozessführung gewährleistet ist. Als Folge davon sinkt der Umsatz und/oder die Selektivität und damit verbunden auch die Ausbeute an werthaltigen Zielprodukten wie kurzkettigen Olefinen. Ferner kommt es zu starken lokalen Erwärmungen insbesondere auch in den Katalysatorzonen, den sogenannten Hotspots, und hierdurch bedingt zu Katalysatorschädigungen, einer vorzeitigen Katalysatordesaktivierung und infolge dessen einer Abnahme der Selektivität bezogen auf das erwünschte Produkt.

Ein Nachteil der in der EP 2032245 B1 gelehrten Reaktionsführung besteht jedoch darin, dass das verwendete Dampf-/Flüssigkeitsverteilungssystem für die Zufuhr des oxygenathaltigen Eduktgemischs zu den Reaktionszonen ein komplexes und kostspieliges System darstellt. Es umfasst viele Instrumente, Rohre, Kompensatoren und Düsen und ist daher empfindlich gegen Fehlbedienungen. Die korrekte Bedienung erfordert daher ein erhebliches Maß an Fähigkeiten und Schulungsaufwand, damit das Betriebsteam das Anfahren der Anlage, das Herunterfahren und den Wechsel des Betriebsmodus vom Normalbetrieb zur Regenerierung des Katalysators sicher beherrscht.

Zu seinem sicheren und störungsfreien Betrieb erfordert es zudem einen hohen Druckabfall im gesamten System für die Zerstäubung und gleichmäßige Verteilung bei unterschiedlichen Flussraten des Eduktgemischs. Dies begrenzt die Betriebsflexibilität des Systems. Ferner werden die Kosten für die stromaufwärts angeordneten Ausrüstungsteile unnötig hoch, da aufgrund des vergleichsweise hohen Druckverlustes über das verwendete Dampf-/Flüssigkeitsverteilungssystem das Druckniveau im dem OTO-Reaktor vorgeschalteten DME-Reaktor und den zuführenden Ausrüstungsteilen ebenfalls relativ hoch ist, was die Wandstärken und Kosten erhöht.

Aus der europäischen Patentanmeldung EP 2760809 A1 ist ein Verfahren bekannt, in dem Kohlenwasserstoffe enthaltendes Rückführgas mit gereinigtem Dimethylether und Wasserdampf gemischt und anschließend auf die Reaktionssektionen eines mehrstufigen OTO-Reaktors aufgegeben wird. Durch den hohen Reinheitsgrad des Dimethylethers und der damit verbundenen Umstellung auf reine Gaszufuhr kann auf eine Zugabe von Wasser und/oder Oxygenaten in flüssiger Form zur Kühlung verzichtet werden. Allerdings ist die Aufreinigung des Dimethylethers durch Abtrennung des nicht umgesetzten Methanols und des durch die DME Bildungsreaktion gebildeten Wassers sehr aufwendig. Nachteilig ist überdies, dass sehr hohe Reinheitsgrade des verwendeten Dimethylethers erforderlich sind, wodurch der Energiebedarf des Verfahrens wieder steigt.

### Beschreibung der Erfindung

Insgesamt ist daher festzustellen, dass weiterhin Bedarf an einem einfachen, betriebssicheren Verfahren zur Herstellung von Olefinen durch Umsetzung eines Oxygenate enthaltenden Eduktgemischs in einem mehrstufigen Oxygenat-zu-Olefin-(OTO)-Synthesereaktor mit einem geringen Energiebedarf existiert. Die Aufgabe der Erfindung ist es daher, ein solches Verfahren und eine korrespondierende Anlage bereitzustellen.

Diese Aufgabe wird im Wesentlichen durch ein Verfahren mit den Merkmalen des Anspruchs 1 gelöst. Weitere, insbesondere bevorzugte Ausgestaltungen des erfindungsgemäßen Verfahrens finden sich in den Unteransprüchen.

### Erfindungsgemäßes Verfahren:

Verfahren zur Herstellung eines Olefine enthaltenden Kohlenwasserstoffproduktes, umfassend Ethylen und Propylen, durch Umsetzung eines Oxygenate enthaltenden Eduktgemischs, das in mehrere Eduktgemisch-Teilströme aufgeteilt wird, in einem mehrstufigen Oxygenat-zu-Olefin-(OTO)-Synthesereaktor, umfassend folgende Schritte:
(a) Bereitstellen des mehrstufigen OTO-Synthesereaktors mit mehreren, hintereinander geschalteten, in Fluidverbindung miteinander stehenden Reaktionssektionen, umfassend eine erste Reaktionssektion und mindestens eine nachfolgende Reaktionssektion, die jeweils Katalysatorzonen mit festen, für die OTO-Synthese aktiven und selektiven Katalysatoren enthalten, wobei stromaufwärts jeder Katalysatorzone eine Zugabevorrichtung für einen Eduktgemisch-Teilstrom angeordnet ist und wobei die stromwärts letzte Reaktionssektion in Fluidverbindung mit einer Leitung zum Ausleiten eines OTO-Synthesereaktorprodukts steht,
(b) Einleiten eines Eduktgemisch-Teilstroms in jede Reaktionssektion über die jeweilige Zugabevorrichtung, wobei die mindestens eine nachfolgende Reaktionssektion zusätzlich mit dem Produktstrom der jeweils stromaufwärts angeordneten Reaktionssektion beaufschlagt wird, Einleiten mindestens eines Wasserdampfstroms in mindestens eine Reaktionssektion, Einleiten mindestens eines Rückführstroms in mindestens eine Reaktionssektion,
(c) mindestens teilweises Umsetzen der zugeführten Oxygenate in den Katalysatorzonen unter Oxygenatumsetzungsbedingungen zu Olefinen und weiteren Kohlenwasserstoffen, Ausleiten des OTO-Synthesereaktorprodukts,
(d) Auftrennen des OTO-Synthesereaktorprodukts in einer mehrstufigen, nach thermischen Trennverfahren arbeitenden Aufarbeitungsvorrichtung, wobei mehrere, Kohlenwasserstoffe enthaltende Kohlenwasserstoff-Produktfraktionen erhalten werden,
(e) Ausleiten eines Olefine, insbesondere Ethylen und/oder Propylen, enthaltenden Kohlenwasserstoffprodukts aus der Aufarbeitungsvorrichtung,
(f) Rückführen mindestens eines Teils einer oder mehrerer Kohlenwasserstoff-Produktfraktionen als Rückführstrom oder Rückführströme zum OTO-Synthesereaktor und Einleiten des oder der Rückführströme in mindestens eine Reaktionssektion,
dadurch gekennzeichnet, dass sämtliche Eduktgemisch-Teilströme, Wasserdampfströme und Rückführströme ausschließlich gasförmig bzw. dampfförmig in den OTO-Synthesereaktor eingeleitet werden und dass das Oxygenate enthaltende Eduktgemisch Dimethylether (DME) enthält und in einem Veretherungsreaktor durch katalytische Dehydratisierung von Methanol in der Gasphase erzeugt wird, wobei ein gasförmiges Veretherungsreaktor-Produktgemisch erhalten wird, das DME, Wasserdampf und Methanoldampf umfasst, wobei das gasförmige Veretherungsreaktor-Produktgemisch ohne zusätzlichen Separationsschritt als Eduktgemisch zu dem OTO-Synthesereaktor geleitet wird.

### Erfindungsgemäße Anlage:

Anlage zur Herstellung eines Olefine enthaltenden Kohlenwasserstoffproduktes, umfassend Ethylen und Propylen, durch Umsetzung eines Oxygenate enthaltenden Eduktgemischs, das in mehrere Eduktgemisch-Teilströme aufgeteilt wird, in einem mehrstufigen Oxygenat-zu-Olefin-(OTO)-Synthesereaktor, umfassend folgende Bestandteile:
(a) einen mehrstufigen OTO-Synthesereaktor mit mehreren, hintereinander geschalteten, in Fluidverbindung miteinander stehenden Reaktionssektionen, umfassend eine erste Reaktionssektion und mindestens eine nachfolgende Reaktionssektion, die jeweils Katalysatorzonen mit festen, für die OTO-Synthese aktiven und selektiven Katalysatoren enthalten, wobei stromaufwärts jeder Katalysatorzone eine Zugabevorrichtung für einen Eduktgemisch-Teilstrom angeordnet ist und wobei die stromwärts letzte Reaktionssektion in Fluidverbindung mit einer Leitung zum Ausleiten eines OTO-Synthesereaktorprodukts steht,
(b) Mittel zum Einleiten eines Eduktgemisch-Teilstroms in jede Reaktionssektion über die jeweilige Zugabevorrichtung, Mittel zum Einleiten mindestens eines Wasserdampfstroms in mindestens eine Reaktionssektion, Mittel zum Einleiten mindestens eines Rückführstroms in mindestens eine Reaktionssektion,
(c) Mittel zum Einstellen von Oxygenatumsetzungsbedingungen, Mittel zum Ausleiten des OTO-Synthesereaktorprodukts,
(d) eine mehrstufige, nach thermischen Trennverfahren arbeitende Aufarbeitungsvorrichtung, geeignet zum Auftrennen des OTO-Synthesereaktorprodukts in mehrere, Kohlenwasserstoffe enthaltende Kohlenwasserstoff-Produktfraktionen, Mittel zum Einleiten des OTO-Synthesereaktorprodukts in die Aufarbeitungsvorrichtung,
(e) Mittel zum Ausleiten eines Olefine, insbesondere Ethylen und/oder Propylen, enthaltenden Kohlenwasserstoffprodukts aus der Aufarbeitungsvorrichtung,
(f) Mittel zum Rückführen mindestens eines Teils einer oder mehrerer in der Aufarbeitungsvorrichtung erhaltener Kohlenwasserstoff-Produktfraktionen als Rückführstrom oder Rückführströme zum OTO-Synthesereaktor und Mittel zum Einleiten des oder der Rückführströme in mindestens eine Reaktionssektion,
dadurch gekennzeichnet, dass alle unter (b) genannten Mittel so ausgestaltet sind, dass sämtliche Eduktgemisch-Teilströme, Wasserdampfströme und Rückführströme gasförmig bzw. dampfförmig in den OTO-Synthesereaktor einleitbar sind und dass ferner ein dem OTO-Synthesereaktor vorgeschalteter Veretherungsreaktor umfasst wird, der so ausgestaltet ist, dass durch katalytische Dehydratisierung von Methanol in der Gasphase ein gasförmiges, Oxygenate enthaltende Eduktgemisch erzeugt werden kann, welches ohne zusätzlichen Separationsschritt zu dem OTO-Synthesereaktor geleitet werden kann.

Die für die Umsetzung von Oxygenaten zu Olefinprodukten erforderlichen Oxygenatumwandlungsbedingungen sind dem Fachmann aus dem Stand der Technik, beispielsweise den eingangs erörterten Druckschriften, bekannt. Es sind diejenigen physikalisch-chemischen Bedingungen, unter denen ein messbarer, bevorzugt ein technisch relevanter Umsatz von Oxygenaten zu Olefinen erzielt wird. Notwendige Anpassungen dieser Bedingungen an die jeweiligen Betriebserfordernisse wird er auf der Grundlage von Routineversuchen vornehmen. Dabei können ggf. offenbarte, spezifische Reaktionsbedingungen als Orientierung dienen, sie sind aber in Bezug auf den Umfang der Erfindung nicht einschränkend zu verstehen.

Thermische Trennverfahren im Sinne der Erfindung sind alle Trennverfahren, die auf der Einstellung eines thermodynamischen Phasengleichgewichtes beruhen. Bevorzugt sind dies die Destillation oder Rektifikation. Grundsätzlich ist aber auch der Einsatz anderer thermischer Trennverfahren denkbar, beispielsweise der Extraktion oder Extraktivdestillation.

Als Aufreinigungsschritt sind im Zusammenhang mit der vorliegenden Erfindung grundsätzlich alle Verfahrensschritte zu betrachten, die sich eines thermischen Trennverfahrens bedienen; bevorzugt wird die Destillation oder Rektifikation verwendet.

Unter Fluidverbindung zwischen zwei Bereichen oder Anlagenteilen wird dabei jegliche Art von Verbindung verstanden, die es ermöglicht, dass ein Fluid, beispielsweise ein Reaktionsprodukt oder eine Kohlenwasserstofffraktion, von dem einen zu dem anderen der beiden Bereiche strömen kann, unbeachtlich etwaiger zwischengeschalteter Bereiche, Bauteile oder benötigter Fördermittel.

Unter einem Mittel wird eine Sache verstanden, die die Erreichung eines Zieles ermöglicht oder dabei behilflich ist. Insbesondere werden unter Mitteln zum Durchführen eines bestimmten Verfahrensschrittes alle diejenigen physischen Gegenstände verstanden, die der Fachmann in Betracht ziehen würde, um diesen Verfahrensschritt durchführen zu können. Beispielsweise wird der Fachmann als Mittel zum Einleiten oder Ausleiten eines Stoffstroms alle Transport- und Fördervorrichtungen, also z. B. Rohrleitungen, Pumpen, Verdichter, Ventile, in Betracht ziehen, die ihm aufgrund seines Fachwissens zur Durchführung dieses Verfahrensschrittes notwendig oder sinnvoll erscheinen.

Unter Oxygenaten werden grundsätzlich alle sauerstoffhaltigen Kohlenwasserstoffverbindungen verstanden, die sich unter Oxygenatumsetzungsbedingungen zu Olefinen, insbesondere zu kurzkettigen Olefinen wie Propylen, und weiteren Kohlenwasserstoffprodukten umsetzen lassen.

Als kurzkettige Olefine werden im Rahmen der vorliegenden Erfindung insbesondere die Olefine verstanden, die bei Umgebungsbedingungen gasförmig vorliegen, beispielsweise Ethylen, Propylen sowie die isomeren Butene 1 -Buten, cis-2-Buten, trans-2-Buten, iso-Buten.

Als höhere Kohlenwasserstoffe werden im Rahmen der vorliegenden Erfindung insbesondere diejenigen Kohlenwasserstoffe verstanden, die bei Umgebungsbedingungen flüssig vorliegen.

Zur Bezeichnung von Kohlenwasserstofffraktionen wird folgende Nomenklatur verwendet: "Cn Fraktion" bezeichnet eine Kohlenwasserstofffraktion, die überwiegend Kohlenwasserstoffe der C-Kettenlänge n, also mit n C-Atomen, enthält. "Cn- Fraktion" bezeichnet eine Kohlenwasserstofffraktion, die überwiegend Kohlenwasserstoffe der C-Kettenlänge n sowie mit niedrigeren C-Kettenlängen enthält. "Cn+ Fraktion" bezeichnet eine Kohlenwasserstofffraktion, die überwiegend Kohlenwasserstoffe der C-Kettenlänge n sowie mit höheren C-Kettenlängen enthält. Aufgrund der verwendeten physikalischen Trennverfahren, beispielsweise der Destillation, ist die Auftrennung hinsichtlich der C-Kettenlänge nicht in der Weise zu verstehen, dass Kohlenwasserstoffe mit anderer Kettenlänge rigoros ausgeschlossen werden. So enthält eine Cn-Fraktion je nach Verfahrensbedingungen des Trennverfahrens immer noch geringe Mengen von Kohlenwasserstoffen mit einer C-Zahl größer als n.

Die genannten Aggregatzustände fest, flüssig und gasförmig bzw. dampfförmig sind immer in Bezug auf die lokalen physikalischen Bedingungen zu verstehen, die bei dem jeweiligen Verfahrensschritt oder in dem jeweiligen Anlagenteil herrschen, sofern nichts anderes angegeben ist. Im Rahmen der vorliegenden Anmeldung sind die Aggregatzustände gasförmig bzw. dampfförmig als synonym zu betrachten. Die Bezeichnung "dampfförmig" dient lediglich zur Verdeutlichung, dass der betreffende Stoff bei Umgebungsbedingungen normalerweise flüssig vorliegt.

Unter einem Auftrennen eines Stoffstroms ist im Zusammenhang mit der vorliegenden Erfindung eine Aufteilung des Stroms in mindestens zwei Teilströme zu verstehen. Wenn nichts anderes angegeben wird, ist davon auszugehen, dass die stoffliche Zusammensetzung der Teilströme derjenigen des Ausgangsstroms entspricht, außer für die Fälle, bei denen dem Fachmann unmittelbar einsichtig ist, dass eine Änderung der stofflichen Zusammensetzung der Teilströme infolge der Bedingungen der Auftrennung zwangsweise auftreten muss.

Unter einer Benzinfraktion ist ein überwiegend, bevorzugt weitgehend vollständig aus höheren Kohlenwasserstoffen bestehendes, unter Umgebungsbedingungen flüssig vorliegendes Stoffgemisch zu verstehen, das geeignet sein kann, als Ottokraftstoff verwendet zu werden.

Unter dem überwiegenden Teil einer Fraktion, eines Stoffstroms etc. ist ein Anteil zu verstehen, der mengenmäßig größer ist als alle anderen jeweils für sich betrachteten Anteile. Insbesondere bei binären Gemischen oder bei der Auftrennung einer Fraktion in zwei Teile ist darunter ein Anteil von mehr als 50 Gew.-% zu verstehen, sofern im konkreten Fall nichts anderes angegeben ist.

Unter der Angabe, dass ein Stoffstrom überwiegend aus einer Komponente oder Komponentengruppe besteht, ist zu verstehen, dass der Stoffmengenanteil (Molenbruch) oder Massenanteil (Massenbruch) dieser Komponente oder Komponentengruppe mengenmäßig größer ist als alle anderen jeweils für sich betrachteten Anteile anderer Komponenten oder Komponentengruppen in dem Stoffstrom. Insbesondere bei binären Gemischen ist darunter ein Anteil von mehr als 50 % zu verstehen. Sofern im konkreten Fall nichts anderes angegeben ist, wird hierbei der Massenanteil (Massenbruch) zugrunde gelegt.

Unter der Angabe, dass Stoffströme ausschließlich gasförmig bzw. dampfförmig in bestimmte Bereiche, beispielsweise den OTO-Synthesereaktor, eingeleitet werden, ist zu verstehen, dass entweder keinerlei Flüssigkeitsbestandteile im eingeleiteten Stoffstrom vorhanden sind oder dass maximal geringe Anteile feinster, gasgetragener Flüssigkeitströpfchen wie Aerosole im Einleitungsstrom vorhanden sind. Ferner bezieht sich diese Angabe im Sinne der Erfindung auf den stationären Zustand des Verfahrens bzw. der Anlage. Es ist nicht auszuschließen, dass während Übergangszuständen, wie beispielsweise beim Anfahren oder Abfahren der Anlage, durch Kondensation oder Flüssigkeitsmitriss während zeitlich begrenzter Betriebsabschnitte auch Flüssigkeitsanteile in die Reaktionssektionen gelangen können.

Druckangaben verstehen sich in bar, absolut, abgekürzt bar(a), sofern im betreffenden Zusammenhang nichts anderes angegeben ist.

Der Erfindung liegt die Erkenntnis zugrunde, dass das Zuführ- und Verteilungssystem des Oxygenate enthaltenden Eduktgemischs erheblich vereinfacht werden kann, wenn alle Eduktgemisch-Teilströme, Wasserdampfströme und Rückführströme ausschließlich gasförmig bzw. dampfförmig in den OTO-Synthesereaktor eingeleitet werden. Bezüglich des vom DME-Reaktor zum OTO-Reaktor geführten Eduktgemischs wird dies dadurch erreicht, dass das gesamte DME-Reaktorprodukt gasförmig zum OTO-Reaktor geführt und diesem aufgegeben wird, ohne dass zuvor eine Kühlung und/oder eine Teilkondensation und Auftrennung in eine an DME angereicherte Gasphase und eine an DME abgereicherte, nicht umgesetztes Methanol und Wasser enthaltende Flüssigphase erfolgt, die getrennt zum OTO-Reaktor geführt und diesem aufgegeben werden. In diesem Fall vereinfacht sich das herkömmliche Dampf-/Flüssigkeitsverteilungssystem, das üblicherweise Zweiphasendüsen enthält, zu einem einfachen Gasverteilungssystem ohne solche Düsen. Hierdurch verringert sich der Druckverlust über das Eduktgemisch-Verteilungssystem und es wird Kompressionsenergie eingespart.

Die Verteilung eines Dampfseitenstroms über eine große Querschnittsfläche ist viel einfacher als die Zerstäubung von Flüssigkeit. Aufgrund der geringen Dichte des Dampfes im Vergleich zu einer Flüssigkeit kann eine gleichmäßige Verteilung mit einem Druckabfall über den Öffnungen erreicht werden, der wesentlich geringer ist als der Druckabfall ist, der für die Flüssigkeitsverteilung und Zerstäubung erforderlich ist.

Die Gesamthöhe des Reaktors kann reduziert werden, da innerhalb der Reaktionssektionen keine freie Länge zur Verdampfung von Flüssigkeitströpfchen mehr erforderlich ist. Hierdurch verringern sich die Baukosten einer entsprechenden Anlage und der Platzbedarf für die Aufstellung derselben.

Der Betriebsdruck des stromaufwärts zum OTO-Reaktor geführten, oxygenathaltigen Zufuhrstroms kann abgesenkt werden, da keine Zweiphasendüsen erforderlich sind. Die Zweiphasendüsen in einem OTO-Reaktor gemäß Stand der Technik erfordern einen relativ hohen Zufuhrdruck auf der stromaufwärtigen Seite sowohl für die Zufuhr auf der Gas-/Dampfseite als auch für die Zufuhr auf der Flüssigkeitsseite, um die Zerstäubung zu unterstützen.

Das Betreiben des Seitenzufuhrsystems bei einem niedrigeren Druck ermöglicht eine weitere Abkühlung der oxygenathaltigen Zufuhrstroms zum OTO-Reaktor auf niedrigere Temperaturen als zuvor. Es ist das Ziel, die Zufuhrtemperatur auf der Seite des oxygenathaltigen Dampfs auf einen Wert etwas oberhalb des Taupunkts zu einzustellen, so dass der Dampfdruck des Gemisches bei dieser Betriebstemperatur immer größer als der Betriebsdruck bleibt.

Durch die Abkühlung auf eine niedrigere Temperatur hat der oxygenathaltige Zufuhrstrom eine größere Kapazität, um das heiße Reaktionsprodukt der stromaufwärtigen Reaktionssektion abzukühlen.

Alternativ oder zusätzlich kann die Kühlung der Reaktionssektionen dadurch bewirkt werden, dass mindestens einer, bevorzugt mehreren, meist bevorzugt allen Reaktionssektionen weitere kalte Gasströme zugeführt werden. Eine einfache Möglichkeit besteht in der Verwendung von Prozessdampf, der in einer OTO-Anlage ohnehin zur Verfügung steht, da er einerseits als Verdünnungsdampf erzeugt wird und andererseits in der OTO-Reaktion Wasser in Dampfform als Nebenprodukt gebildet wird.

Günstig ist es ferner, als Kaltgasstrom mindestens einen kohlenwasserstoffhaltigen Stoffstrom, der in der fraktionierenden Aufarbeitung des OTO-Reaktorprodukts beispielsweise durch fraktionierende Destillation bzw. Rektifikation gewonnen wird, zum OTO-Reaktor zurückzuführen und dort mindestens einer, bevorzugt mehreren, meist bevorzugt allen Reaktionssektionen zuzuführen. Dabei werden leichte Kohlenwasserstoff-Rückführströme, insbesondere im C-Zahl-Bereich C₂ bis C₄, bevorzugt, da sie einen niedrigen Taupunkt aufweisen und somit vor der Zugabe zu den Reaktionssektionen besonders tief abgekühlt werden können, wodurch wiederum eine besonders effektive Temperaturlenkung der Reaktionssektionen ermöglicht wird. Aus dem Stand der Technik war es lediglich bekannt, nur der strömungsmäßig ersten Reaktionssektion einen solchen leichten Kohlenwasserstoffstrom als Rückführstrom aufzugeben.

Überraschenderweise wurde festgestellt, dass eine Verteilung eines oder mehrerer leichten Kohlenwasserstoffströme als Rückführströme auf mehrere, bevorzugt alle Reaktionssektionen zu verbesserten Reaktionsbedingungen aufgrund eines verringerten Partialdrucks der Eduktkomponenten in den einzelnen Katalysatorbetten führt. Der verringerte Partialdruck der Reaktanten erhöht die Selektivität der OTO-Reaktion zu begehrten Zielkomponenten wie insbesondere Ethylen und Propylen.

Zusätzlich können der oder die leichten Kohlenwasserstoffströme als Rückführströme mit den zu den Reaktionssektionen geführten oxygenathaltigen Eduktgemisch-Teilströmen und/oder den zu den Reaktionssektionen geführten Dampfströmen vorgemischt werden, bevor sie den Reaktionssektionen aufgegeben werden. Hierdurch verringert sich der Partialdruck, und folglich der Taupunkt, des Wassers bzw. des Methanols, in die Mischströme, so dass auch diese Ströme vor Einleiten in die einzelnen Reaktionssektionen stärker abgekühlt werden können.

### Bevorzugte Ausgestaltungen der Erfindung

Eine bevorzugte Ausgestaltung des erfindungsgemäßen Verfahrens ist dadurch gekennzeichnet, dass alle Reaktionssektionen mit Eduktgemisch-Teilströmen einerseits und mit Wasserdampfströmen und/oder Rückführströmen andererseits beaufschlagt werden. Diese Maßnahmen sorgen für eine besonders gleichmäßige Verteilung der Eduktkomponenten auf die Reaktionssektionen, was zu einer sehr guten Temperaturkontrolle in den Katalysatorzonen führt. Zudem wird der Partialdruck der Reaktanten gleichmäßig auf niedrigem Niveau gehalten, was zu einem verringerten Partialdruck der Eduktkomponenten in den einzelnen Katalysatorzonen führt. Der verringerte Partialdruck der Reaktanten erhöht die Selektivität der OTO-Reaktion zu begehrten Zielkomponenten wie insbesondere Ethylen und Propylen. Als Reaktanten im vorgenannten Sinne werden dabei nicht nur die den Reaktionssektionen zugeführten Oxygenate, sondern auch die über die Rückführströme zu den Reaktionssektionen zurückgeführten Kohlenwasserstoffe, insbesondere Olefine, betrachtet, die ebenfalls zu den oben genannten Zielkomponenten umgesetzt werden können.

In weiterer, bevorzugter Ausgestaltung des erfindungsgemäßen Verfahrens werden mindestens zwei Kohlenwasserstoff-Produktfraktionen als Rückführströme zu dem OTO-Synthesereaktor zurückgeführt und in diesen eingeleitet. Auf diese Weise können die unterschiedlichen Eigenschaften verschiedener Kohlenwasserstoff-Produktfraktionen besser ausgenutzt werden. So eignen sich Kohlenwasserstoff-Produktfraktionen, die niedermolekulare, niedrigsiedende Kohlenwasserstoffe enthalten, wegen ihres niedrigen Taupunkts besser als gasförmige Kühlmittelströme als höhermolekulare, höhersiedende Kohlenwasserstoffe. Andererseits weisen letztere ein höheres Potential als Reaktivkomponenten auf, da insbesondere höhermolekulare Olefine mit C-Zahlen oberhalb von vier besonders leicht mittels katalytischem Cracken an dem OTO-Synthesekatalysator zu niedermolekularen Olefinen wie insbesondere Ethylen und Propylen umgewandelt werden. Allerdings werden auch die Kohlenwasserstoffe in den Produktfraktionen, die niedermolekulare, niedrigsiedende Kohlenwasserstoffe enthalten, teilweise zu niedermolekularen Olefinen umgewandelt, wenn auch zu einem geringeren Grad als olefinhaltige Fraktionen mit höhermolekularen, höhersiedenden Kohlenwasserstoffen.

Besonders bevorzugt ist es, wenn in die erste Reaktionssektion eine überwiegend C₂ bis C₈ Kohlenwasserstoffe enthaltende Kohlenwasserstoff-Produktfraktion als Rückführstrom eingeleitet wird. Diese Fraktion weist sowohl gute Kühlmitteleigenschaften als auch einen hohen Anteil an höhermolekularen Komponenten wie Olefinen als Reaktivkomponenten auf. Das Einleiten dieser Fraktion in die erste Reaktionssektion ist besonders günstig, da somit die Verweilzeit dieser Fraktion im OTO-Reaktor maximiert wird, so dass eine besonders weitgehende Umsetzung der Reaktivkomponenten zu niedermolekularen Olefinen wie insbesondere Ethylen und Propylen ermöglicht wird.

In Weiterbildung der beiden zuvor erläuterten, besonderen Ausgestaltungen des erfindungsgemäßen Verfahrens wird in die mindestens eine nachfolgende Reaktionssektion ausschließlich eine überwiegend C₂ bis C₄ Kohlenwasserstoffe enthaltende Kohlenwasserstoff-Produktfraktion als Rückführstrom eingeleitet. Hierdurch werden die guten Kühlmitteleigenschaften der niedermolekularen, niedrigsiedenden Kohlenwasserstoffe besonders effektiv ausgenutzt, während gleichzeitig die Verweilzeit der überwiegend C₂ bis C₈ Kohlenwasserstoffe enthaltende Kohlenwasserstoff-Produktfraktion im OTO-Reaktor maximiert wird, so dass eine besonders weitgehende Umsetzung der Reaktivkomponenten zu niedermolekularen Olefinen wie insbesondere Ethylen und Propylen ermöglicht wird.

Eine weitere, bevorzugte Ausgestaltung des erfindungsgemäßen Verfahrens sieht vor, dass der Druckverlust über eine Zugabevorrichtung für einen Eduktgemisch-Teilstrom weniger als 5 bar(a), bevorzugt weniger als 3 bar(a) beträgt. Betriebserfahrungen und weitere Untersuchungen haben gezeigt, dass diese Druckverluste deutlich unter denjenigen liegen, die bei der Dosierung von flüssig/gasförmigen Oxygenatgemischen mittels Zweistoffdüsen in Verfahren gemäß Stand der Technik auftreten. Dies ermöglicht niedrigere Drücke in dem DME-Reaktor und den ihm vorgeschalteten Ausrüstungsteilen und eröffnet so die Möglichkeit eines kostengünstigeren Designs.

In einem weiteren Aspekt ist das erfindungsgemäße Verfahren dadurch gekennzeichnet, dass der Mengenstrom des Rückführstroms und/oder der Mengenstrom des Wasserdampfs für mindestens zwei Reaktionssektionen separat gesteuert oder geregelt wird. Auf diese Weise wird ein besonders flexibler Betrieb des OTO-Synthesereaktors ermöglicht und thermische Fluktuationen können gut ausgeglichen werden.

Eine weitere, bevorzugte Ausgestaltung des erfindungsgemäßen Verfahrens ist dadurch gekennzeichnet, dass der Oxygenat-Partialdruck innerhalb der Katalysatorstufe zwischen 0,1 und 0,5 bar(a) liegt. Untersuchungen haben gezeigt, dass durch Einstellen dieser Oxygenat-Partialdrücke eine besonders günstige Reaktorproduktivität erhalten wird, da somit ein Kompromiss zwischen einer hohen Selektivität für kurzkettige Olefine wie Ethylen und Propylen einerseits (begünstigt durch einen möglichst niedrigen Oxygenat-Partialdruck) und einem hohen Oxygenat-Durchsatz andererseits (begünstigt durch einen möglichst hohen Oxygenat-Partialdruck) erhalten wird.

In einem weiteren Aspekt ist das erfindungsgemäße Verfahren dadurch gekennzeichnet, dass das Oxygenate enthaltende Eduktgemisch Dimethylether (DME) enthält und in einem Veretherungsreaktor durch katalytische Dehydratisierung von Methanol in der Gasphase erzeugt wird, wobei ein gasförmiges Veretherungsreaktor-Produktgemisch erhalten wird, das DME, Wasserdampf und Methanoldampf umfasst, wobei das gasförmige Veretherungsreaktor-Produktgemisch ohne zusätzlichen Separationsschritt als Eduktgemisch zu dem OTO-Synthesereaktor geleitet wird. Vorteilhaft ist es dabei, dass keine Auftrennung des Veretherungsreaktor-Produktgemischs in eine Gasphase und eine Flüssigphase erfolgt, die separat zum OTO-Synthesereaktor geleitet und diesem aufgegeben werden müssen. Hierdurch wird Kühlenergie eingespart, es entfällt eine entsprechende Abscheidevorrichtung und das Leitungssystem wird vereinfacht.

In einem weiteren Aspekt ist das erfindungsgemäße Verfahren dadurch gekennzeichnet, dass das Oxygenate enthaltende Eduktgemisch Dimethylether (DME) enthält und in einem Veretherungsreaktor durch katalytische Dehydratisierung von Methanol in der Gasphase erzeugt wird, wobei ein gasförmiges Veretherungsreaktor-Produktgemisch erhalten wird, das DME, Wasserdampf und Methanoldampf umfasst, wobei das gasförmige Veretherungsreaktor-Produktgemisch ohne zusätzlichen Separationsschritt als Eduktgemisch zu dem OTO-Synthesereaktor geleitet wird und wobei das Oxygenate enthaltende Eduktgemisch einen DME-Gehalt zwischen 50 und 70 Gew.-%, bevorzugt zwischen 55 und 60 Gew.-% aufweist. Untersuchungen haben gezeigt, dass diese Oxygenatgehalte im Eduktgemisch besonders gut im stromabwärts nachfolgenden OTO-Synthesereaktor verarbeitet werden können.

In einem weiteren Aspekt ist das erfindungsgemäße Verfahren dadurch gekennzeichnet, dass das Oxygenate enthaltende Eduktgemisch Dimethylether (DME) enthält und in einem Veretherungsreaktor durch katalytische Dehydratisierung von Methanol in der Gasphase erzeugt wird, wobei ein gasförmiges Veretherungsreaktor-Produktgemisch erhalten wird, das DME, Wasserdampf und Methanoldampf umfasst, wobei das gasförmige Veretherungsreaktor-Produktgemisch ohne zusätzlichen Separationsschritt als Eduktgemisch zu dem OTO-Synthesereaktor geleitet wird und wobei der Absolutdruck des Oxygenate enthaltenden Eduktgemischs vor dem Einleiten in den OTO-Synthesereaktor weniger als 7 bar(a), bevorzugt weniger als 6 bar(a) beträgt und die Temperatur des Oxygenate enthaltenden Eduktgemischs so eingestellt wird, dass sie um mindestens 5 °C, bevorzugt um mindestens 10 °C über dem Taupunkt bei diesem Druck liegt. Untersuchungen haben gezeigt, dass unter diesen Verfahrensbedingungen ein langzeitbeständiger, stabiler Betrieb des Verfahrens ohne vorzeitige Katalysatordesaktivierung und bei gleichzeitig guter Ausbeute an niedermolekularen Olefinen wie insbesondere Ethylen und Propylen ermöglicht wird.

In einem weiteren Aspekt ist das erfindungsgemäße Verfahren dadurch gekennzeichnet, dass das Oxygenate enthaltende Eduktgemisch Dimethylether (DME) enthält und in einem Veretherungsreaktor durch katalytische Dehydratisierung von Methanol in der Gasphase erzeugt wird, wobei ein gasförmiges Veretherungsreaktor-Produktgemisch erhalten wird, das DME, Wasserdampf und Methanoldampf umfasst, wobei das gasförmige Veretherungsreaktor-Produktgemisch ohne zusätzlichen Separationsschritt als Eduktgemisch zu dem OTO-Synthesereaktor geleitet wird und wobei der Absolutdruck des Oxygenate enthaltenden Eduktgemischs vor dem Einleiten in den OTO-Synthesereaktor weniger als 7 bar(a), bevorzugt weniger als 6 bar(a) beträgt und die Temperatur des Oxygenate enthaltenden Eduktgemischs mindestens 140 °C, bevorzugt mindestens 150 °C beträgt. Untersuchungen haben gezeigt, dass unter diesen Verfahrensbedingungen ein besonders langzeitbeständiger, stabiler Betrieb des Verfahrens ohne vorzeitige Katalysatordesaktivierung und bei gleichzeitig sehr guter Ausbeute an niedermolekularen Olefinen wie insbesondere Ethylen und Propylen ermöglicht wird.

In einem weiteren Aspekt ist das erfindungsgemäße Verfahren dadurch gekennzeichnet, dass eine erste und fünf nachfolgende Reaktionssektionen vorhanden sind.

In einem weiteren Aspekt ist das erfindungsgemäße Verfahren dadurch gekennzeichnet, dass die Umsetzung im OTO-Synthesereaktor bei Temperaturen von 300 bis 600 °C, bevorzugt bei Temperaturen von 360 bis 550 °C, meist bevorzugt bei Temperaturen von 400 bis 500 °C durchgeführt wird.

In einem weiteren Aspekt ist das erfindungsgemäße Verfahren dadurch gekennzeichnet, dass die Umsetzung im OTO-Synthesereaktor bei Drücken von 0,1 bis 20 bar, absolut, bevorzugt bei Drücken von 0,5 bis 5 bar, absolut, meist bevorzugt bei Drücken von 1 bis 3 bar, absolut, durchgeführt wird.

In einem weiteren Aspekt ist das erfindungsgemäße Verfahren dadurch gekennzeichnet, dass die Katalysatorzonen in den Reaktionssektionen einen körnigen, formselektiven Zeolith-Katalysator des Pentasiltyps, vorzugsweise ZSM-5, in Form eines Festbettes enthalten.

In einem besonderen Aspekt der erfindungsgemäßen Anlage sind alle Reaktionssektionen mit Mitteln zum Einleiten von Eduktgemisch-Teilströmen einerseits und mit Mitteln zum Einleiten von Wasserdampfströmen und/oder Rückführströmen andererseits ausgestattet. Diese Ausstattungsmerkmale sorgen für eine besonders gleichmäßige Verteilung der Eduktkomponenten auf die Reaktionssektionen, was zu einer sehr guten Temperaturkontrolle in den Katalysatorzonen führt. Zudem wird der Partialdruck der Reaktanten gleichmäßig auf niedrigem Niveau gehalten, was zu einem verringerten Partialdruck der Eduktkomponenten in den einzelnen Katalysatorzonen führt. Der verringerte Partialdruck der Reaktanten erhöht die Selektivität der OTO-Reaktion zu begehrten Zielkomponenten wie insbesondere Ethylen und Propylen. Als Reaktanten im vorgenannten Sinne werden dabei nicht nur die den Reaktionssektionen zugeführten Oxygenate, sondern auch die über die Rückführströme zu den Reaktionssektionen zurückgeführten Kohlenwasserstoffe, insbesondere Olefine, betrachtet, die ebenfalls zu den oben genannten Zielkomponenten umgesetzt werden können.

Bevorzugt umfasst die erfindungsgemäße Anlage eine Aufarbeitungsvorrichtung mit mehreren Trennstufen, in denen unterschiedliche Kohlenwasserstofffraktionen gewonnen werden, wobei ferner mindestens zwei Rückführleitungen umfasst werden, die von unterschiedlichen Trennstufen zu dem OTO-Synthesereaktor zurückführen und die mit unterschiedlichen Mitteln zum Einleiten von Rückführströmen in die Reaktionssektionen verbunden sind. Auf diese Weise wird es ermöglicht, dass mindestens zwei Kohlenwasserstoff-Produktfraktionen als Rückführströme zu dem OTO-Synthesereaktor zurückgeführt und in diesen eingeleitet werden. Hierdurch können die unterschiedlichen Eigenschaften verschiedener Kohlenwasserstoff-Produktfraktionen besser ausgenutzt werden. So eignen sich Kohlenwasserstoff-Produktfraktionen, die niedermolekulare, niedrigsiedende Kohlenwasserstoffe enthalten, wegen ihres niedrigen Taupunkts besser als gasförmige Kühlmittelströme als höhermolekulare, höhersiedende Kohlenwasserstoffe. Andererseits weisen letztere ein höheres Potential als Reaktivkomponenten auf, da insbesondere höhermolekulare Olefine mit C-Zahlen oberhalb von vier besonders leicht mittels katalytischem Cracken an dem OTO-Synthesekatalysator zu niedermolekularen Olefinen wie insbesondere Ethylen und Propylen umgewandelt werden. Allerdings werden auch die Kohlenwasserstoffe in den Produktfraktionen, die niedermolekulare, niedrigsiedende Kohlenwasserstoffe enthalten, teilweise zu niedermolekularen Olefinen umgewandelt, wenn auch zu einem geringeren Grad als olefinhaltige Fraktionen mit höhermolekularen, höhersiedenden Kohlenwasserstoffen.

Bei der zuletzt erörterten Ausgestaltung wird es besonders bevorzugt, wenn eine erste Trennstufe über eine erste Rückführleitung mit der ersten Reaktionssektion verbunden ist und dass eine zweite Trennstufe über eine zweite Rückführleitung mit mindestens einer nachfolgenden Reaktionssektion verbunden ist. Hierdurch wird es insbesondere ermöglicht, dass in die erste Reaktionssektion über die erste Rückführleitung eine überwiegend C₂ bis C₈ Kohlenwasserstoffe enthaltende Kohlenwasserstoff-Produktfraktion als Rückführstrom eingeleitet wird und dass in die mindestens eine nachfolgende Reaktionssektion über die zweite Rückführleitung ausschließlich eine überwiegend C₂ bis C₄ Kohlenwasserstoffe enthaltende Kohlenwasserstoff-Produktfraktion als Rückführstrom eingeleitet wird. Hierdurch werden die guten Kühlmitteleigenschaften der niedermolekularen, niedrigsiedenden Kohlenwasserstoffe in C-Zahl-Bereich von C₂ bis C₄ besonders effektiv ausgenutzt, während gleichzeitig die Verweilzeit der überwiegend C₂ bis C₈ Kohlenwasserstoffe enthaltende Kohlenwasserstoff-Produktfraktion im OTO-Reaktor maximiert wird, so dass eine besonders weitgehende Umsetzung der Reaktivkomponenten zu niedermolekularen Olefinen wie insbesondere Ethylen und Propylen ermöglicht wird.

Ein weiterer Aspekt der erfindungsgemäßen Anlage ist dadurch gekennzeichnet, dass ferner ein dem OTO-Synthesereaktor vorgeschalteter Veretherungsreaktor umfasst wird, der so ausgestaltet ist, dass durch katalytische Dehydratisierung von Methanol in der Gasphase ein gasförmiges, Oxygenate enthaltende Eduktgemisch erzeugt werden kann, welches ohne zusätzlichen Separationsschritt zu dem OTO-Synthesereaktor geleitet werden kann. Vorteilhaft ist es dabei, dass keine Auftrennung des Veretherungsreaktor-Produktgemischs in eine Gasphase und eine Flüssigphase erfolgt, die separat zum OTO-Synthesereaktor geleitet und diesem aufgegeben werden müssen. Hierdurch wird Kühlenergie eingespart, es entfällt eine entsprechende Abscheidevorrichtung und das Leitungssystem wird vereinfacht.

### Ausführungsbeispiel

Weitere Merkmale, Vorteile und Anwendungsmöglichkeiten der Erfindung ergeben sich auch aus der nachfolgenden Beschreibung von Ausführungs- und Zahlenbeispielen und der Zeichnungen. Dabei bilden alle beschriebenen und/oder bildlich dargestellten Merkmale für sich oder in beliebiger Kombination den Gegenstand der Erfindung, unabhängig von ihrer Zusammenfassung in den Ansprüchen oder deren Rückbezug.

Es zeigen:
- Fig. 1: eine schematische Darstellung einer beispielhaften Ausgestaltung des erfindungsgemäßen Verfahrens bzw. der erfindungsgemäßen Anlage,
- Fig. 2: eine schematische Detaildarstellung des OTO-Synthesereaktors mit dem zugehörigen Feedverteilungssystem in beispielhafter Ausgestaltung.

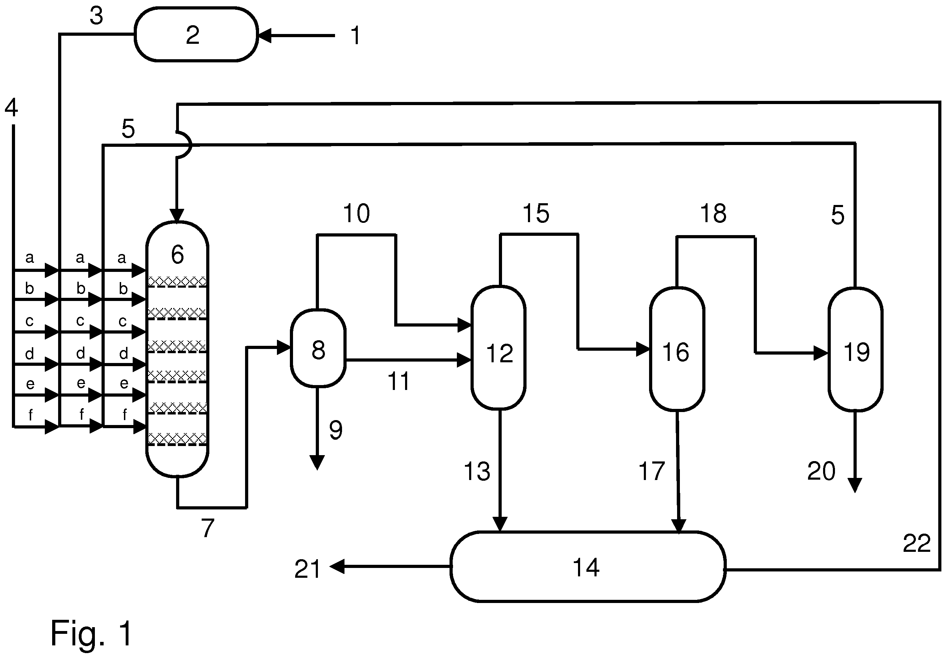

Fig. 1 zeigt schematisch eine beispielhafte Ausgestaltung des erfindungsgemäßen Verfahrens bzw. der erfindungsgemäßen Anlage zur Herstellung eines Olefine enthaltenden Kohlenwasserstoffproduktes, umfassend insbesondere die kurzkettigen Olefine Ethylen und Propylen als Wertprodukte, durch Umsetzung eines Oxygenate enthaltenden Eduktgemischs. Zur Erzeugung des Oxygenate enthaltenden Eduktgemischs wird zunächst Methanoldampf, optional gemeinsam mit Wasserdampf als Verdünnungsmedium, über Leitung 1 dem Dehydratisierungsreaktor (DME-Reaktor) 2 aufgegeben, der mit einer Festbett-Schüttung eines kommerziell erhältlichen Dehydratisierungskatalysators befüllt wurde. An diesem Katalysator erfolgt eine heterogen-katalysierte Teilumsetzung des Methanols unter dem Fachmann bekannten Dehydratisierungsbedingungen zu Dimethylether (DME).

Kennzeichnend für die vorliegende Erfindung ist es, dass das erhaltene, gasförmige Produktgemisch des Dehydratisierungsreaktors, das neben DME auch nicht umgesetztes Methanol sowie Wasserdampf enthält, ohne Abkühlung und Phasentrennung, sondern weiterhin gasförmig mittels Leitung 3 direkt auf den OTO-Synthesereaktor 6 aufgegeben wird, der im vorliegenden Fall sechs Reaktionssektionen umfasst. Die Aufteilung in sechs Eduktgemisch-Teilströme sowie deren Verteilung auf die sechs Reaktionssektionen erfolgt mittels des Leitungssystems 3a bis 3f. Zusätzlich kann über das Leitungssystem 4a bis 4f Wasserdampf herangeführt und ebenfalls auf die sechs Reaktionssektionen verteilt werden. Schließlich wird über das Leitungssystem 5a bis 5f auch ein überwiegend C₂ Kohlenwasserstoffe enthaltender Gasstrom zum OTO-Synthesereaktor zurückgeführt und auf die sechs Reaktionssektionen verteilt. Das in Fig. 1 gezeigte Gasverteilersystem ist dabei rein schematisch zu verstehen. In besonderen Ausgestaltungen können die einzelnen Gasarten Eduktgemisch - Wasserdampf - Kohlenwasserstoff-Rückführstrom entweder separat oder vorgemischt den Reaktionssektionen aufgegeben werden. Dabei ist eine Vormischung der Gasströme zu bevorzugen, da hierdurch der Partialdruck der Reaktivkomponenten abgesenkt wird, was zu einer verbesserten Temperaturführung des OTO-Synthesereaktors und einer verbesserten Selektivität zu kurzkettigen Olefinen führt. Möglich sind dabei Betriebsarten des Reaktors, bei denen dieser entweder mit oxygenathaltigem Eduktgemisch und Wasserdampf als Verdünnungsmittel oder mit oxygenathaltigem Eduktgemisch und einem Kohlenwasserstoff-Rückführstrom als Verdünnungsmittel oder mit oxygenathaltigem Eduktgemisch und gleichzeitig Wasserdampf und einem Kohlenwasserstoff-Rückführstrom als Verdünnungsmittel beaufschlagt wird. Die letztgenannte Betriebsart ist insbesondere dann bevorzugt, wenn der Wasserdampfgehalt in Leitung 3 und die Menge des Kohlenwasserstoff-Rückführstrom noch nicht ausreichen, um eine befriedigende Temperaturkontrolle und Partialdruckeinstellung auf den Reaktionssektionen zu ermöglichen. Sie bietet die größte Flexibilität unter den erörterten Betriebsarten.

Als besondere Ausführungsform der Erfindung kann auch zu einer oder mehreren Reaktionssektionen Wasserdampf und Kohlenwasserstoff geführt werden, wobei der Oxygenatgehalt bis auf einen kleinen Wert, im Extremfall bis auf Null reduziert wird. Hierdurch wird der Umsatz von spezifischen Rückführströmen optimiert oder es können auch kohlenwasserstoffhaltige Ströme aus anderen Verfahren eingebunden werden. Insbesondere wird es bevorzugt, wenn diese Eduktgemisch-Teilströme mit reduziertem oder bis auf Null reduzierten Oxygenatgehalt zu den stromabwärts angeordneten Reaktionssektionen des OTO-Reaktors, besonders bevorzugt zu der letzten Reaktionssektion zugegeben werden.

Die Beaufschlagung der ersten Reaktionssektion mit C₂ Kohlenwasserstoffen über den Leitungsweg 5a kann wahlweise auch entfallen, da der ersten Reaktionssektion bereits über Leitung 22 ein Kohlenwasserstoff-Rückführstrom zugeführt wird.

Die Umsetzung der Oxygenate und Kohlenwasserstoff-Reaktivkomponenten in den Reaktionssektionen des OTO-Synthesereaktors erfolgt unter dem Fachmann bekannten und aus dem einschlägigen Schrifttum ersichtlichen Oxygenatumsetzungsbedingungen. Hierzu sind die Reaktionssektionen mit Katalysatorzonen ausgestattet, die mit Festbett-Schüttungen eines kommerziell erhältlichen Olefinsynthesekatalysators ausgestattet sind.

Das Produktgemisch des OTO-Synthesereaktors wird über Leitung 7 aus diesem ausgeleitet und der mehrstufigen Produktaufarbeitung zugeführt, die in Fig. 1 nur stark schematisch dargestellt ist und nachfolgend nur soweit erläutert wird, wie es für das Verständnis der vorliegenden Erfindung erforderlich ist. Zunächst erfolgt in der Quenchstufe 8 eine Abkühlung des Produktgemischs unter den Taupunkt und nachfolgend eine Phasentrennung in eine wässrige Phase, die über Leitung 9 abgeführt wird, sowie in eine gasförmige und in eine flüssige, jeweils überwiegend Kohlenwasserstoffe enthaltende Phase, die über Leitungen 10 und 11 aus der Quenchstufe abgeführt und beide einer als Debutanizer bezeichneten Destillationskolonne 12 aufgegeben werden.

In der Debutanizer-Destillationskolonne 12 erfolgt die Auftrennung der über Leitungen 10 und 11 herangeführten Kohlenwasserstoffströme mittels fraktionierter Destillation. Als Sumpfprodukt wird dabei eine Kohlenwasserstofffraktion aus der Kolonne 12 ausgeleitet, die Kohlenwasserstoffe mit vier und mehr Kohlenstoffatomen enthält (C₄₊ Fraktion). Sie wird über Leitung 13 einer Aufarbeitungsvorrichtung für schwere Kohlenwasserstofffraktionen 14 zugeführt. In dieser erfolgt mittels mehrerer Trennoperationen, beispielsweise mehrstufiger Destillation, Extraktion, Extraktivdestillation, die weitere Auftrennung des Kohlenwasserstoffgemischs.

Das Kopfprodukt der Kolonne 12 bildet eine Kohlenwasserstofffraktion, die Kohlenwasserstoffe mit vier und weniger Kohlenstoffatomen enthält (C₄₋ Fraktion). Diese Fraktion enthält zudem noch nicht umgesetzte Oxygenate. Sie wird über Leitung 15 aus Kolonne 12 ausgeleitet und einer als Depropanizer bezeichneten Destillationskolonne 16 aufgegeben.

In der Depropanizer-Destillationskolonne 16 erfolgt die Auftrennung des über Leitung 15 herangeführten Kohlenwasserstoffstroms mittels fraktionierter Destillation. Als Sumpfprodukt wird dabei eine Kohlenwasserstofffraktion aus der Kolonne 16 ausgeleitet, die Kohlenwasserstoffe mit vier Kohlenstoffatomen sowie nicht umgesetzte Oxygenate enthält (C₄O Fraktion). Sie wird über Leitung 17 der Aufarbeitungsvorrichtung für schwere Kohlenwasserstofffraktionen 14 zugeführt. In dieser erfolgt mittels mehrerer Trennoperationen, beispielsweise mehrstufiger Destillation, Extraktion, Extraktivdestillation, die weitere Auftrennung des Kohlenwasserstoffgemischs.

Das Kopfprodukt der Kolonne 16 bildet eine Kohlenwasserstofffraktion, die Kohlenwasserstoffe mit drei und weniger Kohlenstoffatomen enthält (C₃₋ Fraktion). Sie wird über Leitung 18 aus Kolonne 16 ausgeleitet und einer als Deethanizer bezeichneten Destillationskolonne 19 aufgegeben.

In der Deethanizer-Destillationskolonne 19 erfolgt die Auftrennung des über Leitung 18 herangeführten Kohlenwasserstoffstroms mittels fraktionierter Destillation. Als Sumpfprodukt wird dabei eine Kohlenwasserstofffraktion aus der Kolonne 19 ausgeleitet, die Kohlenwasserstoffe mit drei Kohlenstoffatomen umfasst und neben Propan demnach auch das Zielprodukt Propylen enthält. Sie wird über Leitung 20 einer nicht gezeigten Aufarbeitungsvorrichtung zugeführt, in der Propan und Propylen destillativ getrennt werden und die optional weitere Aufarbeitungsstufen enthält, so dass das Zielprodukt Propylen rein erhalten werden kann.

Das Kopfprodukt der Kolonne 19 bildet eine Kohlenwasserstofffraktion, die Kohlenwasserstoffe mit zwei und weniger Kohlenstoffatomen enthält (C₂₋ Fraktion). Sie wird über Leitung 5 aus Kolonne 19 ausgeleitet und nach weiteren, optionalen und bildlich nicht dargestellten Aufarbeitungs- oder Konditionierungsschritten in einen Teilstrom aufgetrennt, der über eine bildlich nicht gezeigte Leitung aus dem Verfahren als Spülstrom oder Purgestrom ausgeleitet. Falls dies gewünscht ist, kann aus dem Spülstrom noch durch an sich bekannte Aufarbeitungsschritte Ethylen als Reinprodukt erhalten werden. Aus dem verbleibenden Anteil wird ein kleinerer Teilstrom als Purge abgetrennt und der verbliebene Strom der C₂₋ Fraktion wird über Leitung 5 zum OTO-Synthesereaktor zurückgeführt.

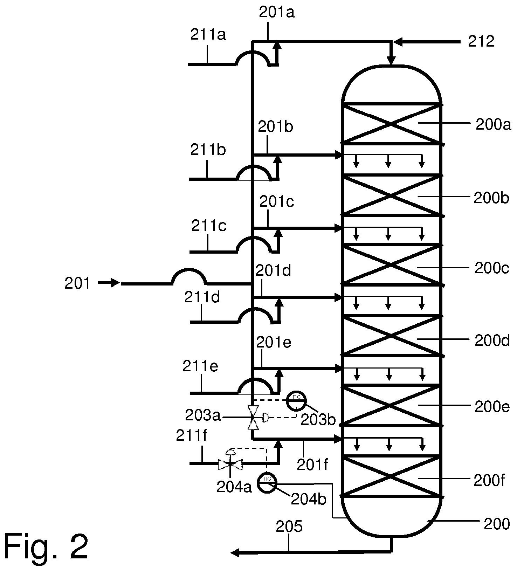

Der in Fig. 2 schematisch dargestellte OTO-Synthesereaktor 200 zur Umsetzung von DME zu Olefinen ist als Festbettreaktor mit mehreren Reaktionssektionen 200a-200f ausgestaltet, die jeweils Zonen eines für die OTO-Synthese reaktiven und selektiven Katalysators enthalten. Es empfiehlt sich, wenigstens drei, bevorzugt wenigstens vier, meist bevorzugt, wie in Fig. 2 dargestellt, sechs Katalysatorstufen vorzusehen. Diese Ausgestaltung des OTO-Synthesereaktor stell einen günstigen Kompromiss dar. Noch mehr Reaktionssektionen würden die pro Sektion freigesetzte Reaktionsenthalpie weiter verringern und wären daher günstig für die Temperaturlenkung des Reaktors; nachteilig wäre aber die steigenden Investitionskosten und der zunehmende Regelungsaufwand.

Die Beaufschlagung mit Dimethylether als Oxygenat erfolgt durch eine Aufteilung des Eduktstroms in Leitung 201 in die einzelnen Eduktteilströme in Leitungen 201a bis 201f. Gleichzeitig wird über Leitungen 211a bis 211f allen Reaktionssektionen ein C2 Kohlenwasserstoffe enthaltendes Rückführgas zugeführt; dies kann, wie anhand von Fig. 1 erläutert, ein Teilstrom des Kopfprodukts des Deethanizers sein. Ferner wird über Leitung 212 der ersten Reaktionssektionen ein C4 bis C6 Kohlenwasserstoffe enthaltendes Rückführgas zugeführt, das durch die Aufarbeitung der Sumpfprodukte des Debutanizers und des Depropanizers gewonnen wird. Letzteres kann noch Anteile an nicht umgesetztem DME enthalten, die ebenfalls zum OTO-Synthesereaktor zurückgeführt werden. Allen dem Reaktor 200 aufgegeben Strömen kann noch Wasserdampf beigemischt werden; zusätzlich oder alternativ kann Wasserdampf einer oder mehreren Reaktionssektionen über bildlich nicht gezeigte Zuleitungen zugegeben werden. Dies empfiehlt sich insbesondere dann, wenn der Wasserdampfstrom aus Gründen der verbesserten Temperaturführung separat von den Eduktteilströmen oder Rückführströmen geregelt werden soll. Entscheidend und kennzeichnend für die Erfindung ist es, dass alle diese Stoffströme dem OTO-Synthesereaktor gasförmig aufgegeben werden. Dies kann beispielsweise dadurch erreicht werden, dass die Temperatur des C2 Kohlenwasserstoffe enthaltenden Rückführgases zwischen 0 und 50 °C und des Wasserdampfes zwischen 100 und 220 °C gewählt wird. Die Temperatur des C4 bis C6 Kohlenwasserstoffe enthaltenden Rückführgases muss aufgrund des Anteils an höhersiedenden Kohlenwasserstoffen höher als diejenige des ersten Rückführgases gewählt werden; entscheidend ist es hierbei, dass die Temperatur sicher oberhalb des Taupunkts liegt, der von der genauen Zusammensetzung der Fraktion abhängt.

Die einzelnen Reaktionssektionen sind hintereinandergeschaltet. Durch die Vermischung des kalten Einsatzgases mit dem aus der vorhergehenden Katalysatorstufe austretenden heißen Produktgas wird letzteres gekühlt und kann so in der folgenden Reaktionsstufe mit dem beigemischten Dimethylether und den Reaktivkomponenten im Rückführgas im gewünschten Temperaturbereich reagieren.

Die Mischung eines Dimethylether enthaltenden Eduktteilstroms und Rückführgas ist in der letzten Stufe 200f exemplarisch dargestellt. Über einen Durchflussregler 203b und das zugeordnete Regelventil 203a wird der Eduktteilstrom so eingestellt, dass die gewünschte Oxygenatmenge in die Reaktionssektion 200f eingespeist wird. Durch den über Leitung 201f zugeführten, kalten Eduktteilstrom wird zwar bereits eine gewisse Kühlung erreicht, wenn sich dieser Strom mit dem Produktstrom aus der vorgeschalteten Reaktionssektion 200e mischt. Zusätzlich kann über Ventil 204a C2 haltiges Rückführgas und/oder Wasserdampf zudosiert werden, so dass über den Temperaturregler 204b auch die gewünschte Solltemperatur des Austrittstroms aus der Reaktionssektion erreicht wird.

Dieses Temperatur- und Reaktionsführungskonzept wird günstigerweise bei allen anderen Reaktionssektionen, zumindest aber bei den Reaktionssektionen 2 bis 6 gleichartig ausgeführt. Die Ein- und Austrittstemperaturen der jeweiligen Stufe sind flexibel und über das Mengenverhältnis der jeweiligen DME- und Recyclingströme einfach einstellbar. Es kann also über den gesamten Reaktor ein für eine maximale Ethylen- und/oder Propylenausbeute optimales Temperaturprofil eingestellt werden.

### Zahlenbeispiele

Im Einzelnen kann ein wie in Fig. 2 dargestellter Reaktor mit den folgenden Einstellungen vorteilhaft betrieben werden:
Über die Reaktionssektionen 200a und 200e des Reaktors kann ein vorgewähltes Temperaturniveau eingestellt und in der nächsten Reaktionssektion die Zusatzkühlung mit Oxygenat, einem überwiegend aus C₂ Kohlenwasserstoffen bestehenden Rückführgas, welche eine bevorzugte Temperatur zwischen 120 und 160 °C aufweist, und/oder Prozessdampf minimiert werden. Die Temperaturen in den Reaktionssektionen 200a und 200e liegen vorzugsweise zwischen 470 und 500 °C. Alle zu den Reaktionssektionen zugegebenen Stoffströme sind gasförmig und wurden so bemessen, dass pro Reaktionssektion nahezu derselbe Temperaturanstieg erhalten wird wie bei einem Verfahren gemäß Stand der Technik mit demselben, sechsstufigen Reaktor, aber zweiphasiger Zuführung des Eduktgemischs gas/flüssig über Zweistoffdüsen.

In der letzten Reaktionssektion 200f wird ein reduzierter Umsatz an DME bzw. möglichst flach verlaufendes Temperaturintervall über der Reaktionssektion eingestellt. Erfindungsgemäß bewegt sich dann das Temperaturprofil in der Reaktionssektion 200f beispielsweise zwischen 480 und 500 °C. Damit findet bei maximaler Temperatur und geringer Neubildung aus DME eine möglichst weitgehende Abreaktion der im Reaktionsgas befindlichen C₂ bis C₄ Olefine zu Propylen statt. Vergleichbare Einstellungen sind bei Ausgestaltung des Reaktors gemäß Stand der Technik nur eingeschränkt möglich, da die Exothermie der entsprechenden Reaktion bei der Anwesenheit von Oxygenaten niedrigere Eintrittstemperaturen erfordert.

Durch das Abkühlen des Oxygenat- und Rückführgasstroms auf 120 bis 160 °C kommt es zu einer effizienten Kühlung des Produktgases bei der Einspeisung des Oxygenat-Rückführgas-Gemisches in die Reaktionssektionen. Bei dem nachfolgend in Tabelle 1 dargestellten Beispiel 1 ergeben sich für eine Verwendung von ca. 84 Gew.-% Ethylen im Rückführgas und ohne Dampfeinspeisung die dort zusammengefassten Prozessdaten hinsichtlich der Kühlung in den einzelnen Reaktionssektionen.

Zur Kühlung kann anstelle des zuvor beschriebenen Rückführgasstroms auch Prozessdampf über eine separate Seiteneinspeisung mit dem Oxygenatstrom vor dem Aufgeben auf die jeweilige Reaktionssektion beigemischt werden, wie nachfolgend in Tabelle 2 als Beispiel 2 dargestellt.

In einer weiteren Ausführungsform der Erfindung kann auch ein Rückführgasstrom und ein Prozessdampfstrom mit einem Oxygenatstrom kombiniert und gemeinsam auf eine Reaktionssektion aufgegeben werden, wie nachfolgend in Tabelle 3 als Beispiel 3 gezeigt wird.

**Tabelle 1: Kühlung der einzelnen Reaktionssektionen mittels DME und C₂ Rückführgas (Beispiel 1)**

| | Kühlbedarf stromaufwärts der Sektion | Kühlung durch DME (gas) | Kühlung durch C₂ Rückführgas |
|---|---|---|---|
| Reaktionssektion #1 | 0% | - | - |
| Reaktionssektion #2 | 100% | 95.3% | 4.7% |
| Reaktionssektion #3 | 100% | 88.9% | 11.1% |
| Reaktionssektion #4 | 100% | 84.0% | 16.0% |
| Reaktionssektion #5 | 100% | 80.4% | 19.6% |
| Reaktionssektion #6 | 100% | 77.6% | 22.4% |

**Tabelle 2: Kühlung der einzelnen Reaktionssektion mittels DME und Prozessdampf (Beispiel 2)**

| | Kühlbedarf stromaufwärts der Sektion | Kühlung durch DME (gas) | Kühlung durch Prozessdampf |
|---|---|---|---|
| Reaktionssektion #1 | 0% | - | - |
| Reaktionssektion #2 | 100% | 89.0% | 11.0% |
| Reaktionssektion #3 | 100% | 84.7% | 15.3% |
| Reaktionssektion #4 | 100% | 81.8% | 18.2% |
| Reaktionssektion #5 | 100% | 79.7% | 20.3% |
| Reaktionssektion #6 | 100% | 78.3% | 21.7% |

Durch die geringeren Eintrittstemperaturen verringern sich zudem die Partialdrücke der einzelnen Reaktanten, wie es nachfolgend in Tabelle 4 für die obigen drei Beispiele zusammengestellt und mit einer Ausführungsform gemäß Stand der Technik verglichen wird.

Unter sonst vergleichbaren Bedingungen kann bei einer auf einem gasförmigen DME-Eduktstrom und gasförmigen Verdünnungsmedien basierten OTO-Anlage eine bis zu 2 % höhere Propylen-Selektivität erhalten werden als bei einer vergleichbaren Anlage gemäß Stand der Technik. Die Selektivitätserhöhung wird durch die gezeigte Verringerung des Partialdrucks der Reaktivkomponenten und ferner dadurch erreicht, dass die Reaktionstemperaturen in den einzelnen Reaktionssektionen durch die erfindungsgemäße Temperaturführung im optimalen Bereich gehalten werden können.

**Tabelle 3: Kühlung der einzelnen Reaktionssektion mittels DME, C₂-Rückführgas und Prozessdampf (Beispiel 3)**

| | Kühlbedarf stromaufwärts der Sektion | Kühlung durch DME (gas) | Kühlung durch C₂ Rückführgas | Kühlung durch Prozessdampf |
|---|---|---|---|---|
| Reaktionssektion #1 | 0% | - | - | - |
| Reaktionssektion #2 | 100% | 87.3% | 5.9% | 6.8% |
| Reaktionssektion #3 | 100% | 81.2% | 12.4% | 6.4% |
| Reaktionssektion #4 | 100% | 76.5% | 17.4% | 6.1% |
| Reaktionssektion #5 | 100% | 73.0% | 21.1% | 5.9% |
| Reaktionssektion #6 | 100% | 70.3% | 23.9% | 5.7% |

**Tabelle 4: Partialdrücke der Reaktanten bei Eintritt und Austritt für einzelne Reaktionssektionen (alle Drücke als bar(a)).**

| P_{Reakt} = Partialdruck Reaktanten | Vergleichsbsp. (St. d. Technik) | Beispiel 1 (Erfindung) | Beispiel 2 (Erfindung) | Beispiel 3 (Erfindung) |
|---|---|---|---|---|
| P_{Reakt} zu Sektion #1 | 0.438 bar | 0.320 bar | 0.486 bar | 0.322 bar |
| P_{Reakt} aus Sektion #1 | 0.357 bar | 0.250 bar | 0.395 bar | 0.251 bar |
| P_{Reakt} zu Sektion #2 | 0.410 bar | 0.318 bar | 0.446 bar | 0.319 bar |
| P_{Reakt} aus Sektion #2 | 0.331 bar | 0.249 bar | 0.359 bar | 0.248 bar |
| P_{Reakt} zu Sektion #3 | 0.381 bar | 0.317 bar | 0.404 bar | 0.317 bar |
| P_{Reakt} aus Sektion #3 | 0.303 bar | 0.246 bar | 0.321 bar | 0.245 bar |
| P_{Reakt} zu Sektion #4 | 0.351 bar | 0.315 bar | 0.363 bar | 0.312 bar |
| P_{Reakt} aus Sektion #4 | 0.274 bar | 0.241 bar | 0.283 bar | 0.239 bar |
| P_{Reakt} zu Sektion #5 | 0.321 bar | 0.309 bar | 0.322 bar | 0.305 bar |
| P_{Reakt} aus Sektion #5 | 0.245 bar | 0.234 bar | 0.246 bar | 0.231 bar |
| P_{Reakt} zu Sektion #6 | 0.292 bar | 0.284 bar | 0.302 bar | 0.297 bar |
| P_{Reakt} aus Sektion #6 | 0.216 bar | 0.210 bar | 0.224 bar | 0.220 bar |

### Bezugszeichenliste

- 1: Leitung
- 2: DME-Reaktor
- 3 - 5: Leitung
- 6: OTO-Synthesereaktor
- 7: Leitung
- 8: Quench
- 9 - 11: Leitung
- 12: Trennkolonne (Debutanizer)
- 13: Leitung
- 14: Aufarbeitungsvorrichtung
- 15: Leitung
- 16: Trennkolonne (Depropanizer)
- 17 - 18: Leitung
- 19: Trennkolonne (Deethanizer)
- 20 - 22: Leitung

- 200: OTO-Synthesereaktor
- 201: Leitung
- 203a, 204a: Regelventil
- 203b: Durchflussmessung
- 204b: Temperaturmessung
- 205: Leitung
- 211 - 212: Leitung

## Patentansprüche

1. Verfahren zur Herstellung eines Olefine enthaltenden Kohlenwasserstoffproduktes, umfassend Ethylen und Propylen, durch Umsetzung eines Oxygenate enthaltenden Eduktgemischs, das in mehrere Eduktgemisch-Teilströme aufgeteilt wird, in einem mehrstufigen Oxygenat-zu-Olefin-(OTO)-Synthesereaktor, umfassend folgende Schritte:
(a) Bereitstellen des mehrstufigen OTO-Synthesereaktors mit mehreren, hintereinander geschalteten, in Fluidverbindung miteinander stehenden Reaktionssektionen, umfassend eine erste Reaktionssektion und mindestens eine nachfolgende Reaktionssektion, die jeweils Katalysatorzonen mit festen, für die OTO-Synthese aktiven und selektiven Katalysatoren enthalten, wobei stromaufwärts jeder Katalysatorzone eine Zugabevorrichtung für einen Eduktgemisch-Teilstrom angeordnet ist und wobei die stromwärts letzte Reaktionssektion in Fluidverbindung mit einer Leitung zum Ausleiten eines OTO-Synthesereaktorprodukts steht,
(b) Einleiten eines Eduktgemisch-Teilstroms in jede Reaktionssektion über die jeweilige Zugabevorrichtung, wobei die mindestens eine nachfolgende Reaktionssektion zusätzlich mit dem Produktstrom der jeweils stromaufwärts angeordneten Reaktionssektion beaufschlagt wird, Einleiten mindestens eines Wasserdampfstroms in mindestens eine Reaktionssektion, Einleiten mindestens eines Rückführstroms in mindestens eine Reaktionssektion,
(c) mindestens teilweises Umsetzen der zugeführten Oxygenate in den Katalysatorzonen unter Oxygenatumsetzungsbedingungen zu Olefinen und weiteren Kohlenwasserstoffen, Ausleiten des OTO-Synthesereaktorprodukts,
(d) Auftrennen des OTO-Synthesereaktorprodukts in einer mehrstufigen, nach thermischen Trennverfahren arbeitenden Aufarbeitungsvorrichtung, wobei mehrere, Kohlenwasserstoffe enthaltende Kohlenwasserstoff-Produktfraktionen erhalten werden,
(e) Ausleiten eines Olefine, insbesondere Ethylen und/oder Propylen, enthaltenden Kohlenwasserstoffprodukts aus der Aufarbeitungsvorrichtung,
(f) Rückführen mindestens eines Teils einer oder mehrerer Kohlenwasserstoff-Produktfraktionen als Rückführstrom oder Rückführströme zum OTO-Synthesereaktor und Einleiten des oder der Rückführströme in mindestens eine Reaktionssektion,
**dadurch gekennzeichnet, dass** sämtliche Eduktgemisch-Teilströme, Wasserdampfströme und Rückführströme ausschließlich gasförmig bzw. dampfförmig in den OTO-Synthesereaktor eingeleitet werden und dass das Oxygenate enthaltende Eduktgemisch Dimethylether (DME) enthält und in einem Veretherungsreaktor durch katalytische Dehydratisierung von Methanol in der Gasphase erzeugt wird, wobei ein gasförmiges Veretherungsreaktor-Produktgemisch erhalten wird, das DME, Wasserdampf und Methanoldampf umfasst, wobei das gasförmige Veretherungsreaktor-Produktgemisch ohne zusätzlichen Separationsschritt als Eduktgemisch zu dem OTO-Synthesereaktor geleitet wird.

2. Verfahren nach Anspruch 1, **dadurch gekennzeichnet, dass** alle Reaktionssektionen mit Eduktgemisch-Teilströmen einerseits und mit Wasserdampfströmen und/oder Rückführströmen andererseits beaufschlagt werden.

3. Verfahren nach Anspruch 1 oder 2, **dadurch gekennzeichnet, dass** mindestens zwei Kohlenwasserstoff-Produktfraktionen als Rückführströme zu dem OTO-Synthesereaktor zurückgeführt und in diesen eingeleitet werden.

4. Verfahren nach Anspruch 3, **dadurch gekennzeichnet, dass** in die erste Reaktionssektion eine überwiegend C₂ bis C₈ Kohlenwasserstoffe enthaltende Kohlenwasserstoff-Produktfraktion als Rückführstrom eingeleitet wird.

5. Verfahren nach Anspruch 3 oder 4, **dadurch gekennzeichnet, dass** in die mindestens eine nachfolgende Reaktionssektion ausschließlich eine überwiegend C₂ bis C₄ Kohlenwasserstoffe enthaltende Kohlenwasserstoff-Produktfraktion als Rückführstrom eingeleitet wird.

6. Verfahren nach einem der vorhergehenden Ansprüche, **dadurch gekennzeichnet, dass** der Druckverlust über eine Zugabevorrichtung für einen Eduktgemisch-Teilstrom weniger als 5 bar(a), bevorzugt weniger als 3 bar(a) beträgt.

7. Verfahren nach einem der vorhergehenden Ansprüche, **dadurch gekennzeichnet, dass** der Mengenstrom des Rückführstroms und/oder der Mengenstrom des Wasserdampfs für mindestens zwei Reaktionssektionen separat gesteuert oder geregelt wird.

8. Verfahren nach einem der vorhergehenden Ansprüche, **dadurch gekennzeichnet, dass** der Oxygenat-Partialdruck innerhalb der Katalysatorstufe zwischen 0,1 und 0,5 bar(a) liegt.

9. Verfahren nach Anspruch 1, **dadurch gekennzeichnet, dass** das Oxygenate enthaltende Eduktgemisch einen DME-Gehalt zwischen 50 und 70 Gew.-%, bevorzugt zwischen 55 und 60 Gew.-% aufweist.

10. Verfahren nach Anspruch 1, **dadurch gekennzeichnet, dass** der Absolutdruck des Oxygenate enthaltenden Eduktgemischs vor dem Einleiten in den OTO-Synthesereaktor weniger als 7 bar(a), bevorzugt weniger als 6 bar(a) beträgt und die Temperatur des Oxygenate enthaltenden Eduktgemischs so eingestellt wird, dass sie um mindestens 5 °C, bevorzugt um mindestens 10 °C über dem Taupunkt bei diesem Druck liegt.

11. Verfahren nach Anspruch 1, **dadurch gekennzeichnet, dass** der Absolutdruck des Oxygenate enthaltenden Eduktgemischs vor dem Einleiten in den OTO-Synthesereaktor weniger als 7 bar(a), bevorzugt weniger als 6 bar(a) beträgt und die Temperatur des Oxygenate enthaltenden Eduktgemischs mindestens 140 °C, bevorzugt mindestens 150 °C beträgt.

12. Anlage zur Herstellung eines Olefine enthaltenden Kohlenwasserstoffproduktes, umfassend Ethylen und Propylen, durch Umsetzung eines Oxygenate enthaltenden Eduktgemischs, das in mehrere Eduktgemisch-Teilströme aufgeteilt wird, in einem mehrstufigen Oxygenat-zu-Olefin-(OTO)-Synthesereaktor, umfassend folgende Bestandteile:
(a) einen mehrstufigen OTO-Synthesereaktor mit mehreren, hintereinander geschalteten, in Fluidverbindung miteinander stehenden Reaktionssektionen, umfassend eine erste Reaktionssektion und mindestens eine nachfolgende Reaktionssektion, die jeweils Katalysatorzonen mit festen, für die OTO-Synthese aktiven und selektiven Katalysatoren enthalten, wobei stromaufwärts jeder Katalysatorzone eine Zugabevorrichtung für einen Eduktgemisch-Teilstrom angeordnet ist und wobei die stromwärts letzte Reaktionssektion in Fluidverbindung mit einer Leitung zum Ausleiten eines OTO-Synthesereaktorprodukts steht,
(b) Mittel zum Einleiten eines Eduktgemisch-Teilstroms in jede Reaktionssektion über die jeweilige Zugabevorrichtung, Mittel zum Einleiten mindestens eines Wasserdampf stroms in mindestens eine Reaktionssektion, Mittel zum Einleiten mindestens eines Rückführstroms in mindestens eine Reaktionssektion,
(c) Mittel zum Einstellen von Oxygenatumsetzungsbedingungen, Mittel zum Ausleiten des OTO-Synthesereaktorprodukts,
(d) eine mehrstufige, nach thermischen Trennverfahren arbeitende Aufarbeitungsvorrichtung, geeignet zum Auftrennen des OTO-Synthesereaktorprodukts in mehrere, Kohlenwasserstoffe enthaltende Kohlenwasserstoff-Produktfraktionen, Mittel zum Einleiten des OTO-Synthesereaktorprodukts in die Aufarbeitungsvorrichtung,
(e) Mittel zum Ausleiten eines Olefine, insbesondere Ethylen und/oder Propylen, enthaltenden Kohlenwasserstoffprodukts aus der Aufarbeitungsvorrichtung,
(f) Mittel zum Rückführen mindestens eines Teils einer oder mehrerer in der Aufarbeitungsvorrichtung erhaltener Kohlenwasserstoff-Produktfraktionen als Rückführstrom oder Rückführströme zum OTO-Synthesereaktor und Mittel zum Einleiten des oder der Rückführströme in mindestens eine Reaktionssektion,
**dadurch gekennzeichnet, dass** alle unter (b) genannten Mittel so ausgestaltet sind, dass sämtliche Eduktgemisch-Teilströme, Wasserdampfströme und Rückführströme gasförmig bzw. dampfförmig in den OTO-Synthesereaktor einleitbar sind und dass ferner ein dem OTO-Synthesereaktor vorgeschalteter Veretherungsreaktor umfasst wird, der so ausgestaltet ist, dass durch katalytische Dehydratisierung von Methanol in der Gasphase ein gasförmiges, Oxygenate enthaltende Eduktgemisch erzeugt werden kann, welches ohne zusätzlichen Separationsschritt zu dem OTO-Synthesereaktor geleitet werden kann.

13. Anlage nach Anspruch 12, **dadurch gekennzeichnet, dass** alle Reaktionssektionen mit Mitteln zum Einleiten von Eduktgemisch-Teilströmen einerseits und mit Mitteln zum Einleiten von Wasserdampfströmen und/oder Rückführströmen andererseits ausgestattet sind.

14. Anlage nach Anspruch 12 oder 13, **dadurch gekennzeichnet, dass** die Aufarbeitungsvorrichtung mehrere Trennstufen umfasst, in denen unterschiedliche Kohlenwasserstofffraktionen gewonnen werden, wobei ferner mindestens zwei Rückführleitungen umfasst werden, die von unterschiedlichen Trennstufen zu dem OTO-Synthesereaktor zurückführen und die mit unterschiedlichen Mitteln zum Einleiten von Rückführströme in die Reaktionssektionen verbunden sind.

15. Anlage nach Anspruch 14, **dadurch gekennzeichnet, dass** eine erste Trennstufe über eine erste Rückführleitung mit der ersten Reaktionssektion verbunden ist und dass eine zweite Trennstufe über eine zweite Rückführleitung mit mindestens einer nachfolgenden Reaktionssektion verbunden ist.

## Claims

1. Process for producing an olefins-containing hydrocarbon product comprising ethylene and propylene by conversion of an oxygenates-containing reactant mixture, which is divided into a plurality of reactant mixture substreams, in a multi-stage oxygenate-to-olefin (OTO) synthesis reactor, comprising the following steps:
(a) providing the multistage OTO synthesis reactor having a plurality of serially connected reaction sections in fluid connection with one another comprising a first reaction section and at least one subsequent reaction section which each contain catalyst zones comprising solid catalysts that are active and selective for OTO synthesis, wherein upstream of each catalyst zone a feeding apparatus for a reactant mixture substream is arranged and wherein the last reaction section in the direction of flow is in fluid connection with a conduit for discharging an OTO synthesis reactor product,
(b) introducing a reactant mixture substream into each reaction section via the respective feeding apparatus, wherein the at least one subsequent reaction section is additionally supplied with the product stream from the respective upstream reaction section, introducing at least one steam stream into at least one reaction section, introducing at least one recycle stream into at least one reaction section,
(c) at least partially converting the supplied oxygenates in the catalyst zones under oxygenate conversion conditions into olefins and further hydrocarbons, discharging the OTO synthesis reactor product,
(d) separating the OTO synthesis reactor product in a multistage workup apparatus operating by means of thermal separation processes to obtain a plurality of hydrocarbons-containing hydrocarbon product fractions,
(e) discharging an olefins-containing, in particular ethylene- and/or propylene-containing, hydrocarbon product from the workup apparatus,
(f) recycling at least a portion of one or more hydrocarbon product fractions to the OTO synthesis reactor as a recycle stream or recycle streams and introducing the recycle stream(s) into at least one reaction section,
**characterized in that** all reactant mixture substreams, steam streams and recycle streams are introduced into the OTO synthesis reactor exclusively in gaseous/vaporous form and **in that** the oxygenates-containing reactant mixture contains dimethyl ether (DME) and is produced in an etherification reactor by catalytic dehydration of methanol in the gas phase to obtain a gaseous etherification reactor product mixture comprising DME, steam and methanol vapour, wherein the gaseous etherification reactor product mixture is sent to the OTO synthesis reactor as reactant mixture without an additional separation step.

2. Process according to Claim 1, **characterized in that** all reaction sections are supplied with reactant mixture substreams on the one hand and with steam streams and/or recycle streams on the other hand.

3. Process according to Claim 1 or 2, **characterized in that** at least two hydrocarbon product fractions are recycled to the OTO synthesis reactor as recycle streams and introduced thereto.

4. Process according to Claim 3, **characterized in that** a hydrocarbon product fraction containing predominantly C₂ to C₈ hydrocarbons is introduced into the first reaction section as a recycle stream.

5. Process according to Claim 3 or 4, **characterized in that** exclusively a hydrocarbon product fraction containing predominantly C₂ to C₄ hydrocarbons is introduced into the at least one subsequent reaction section as a recycle stream.

6. Process according to any of the preceding claims, **characterized in that** the pressure drop over a feeding apparatus for a reactant mixture substream is less than 5 bar(a), preferably less than 3 bar(a).

7. Process according to any of the preceding claims, **characterized in that** the mass flow of the recycle stream and/or the mass flow of the steam is separately controlled or regulated for at least two reaction sections.

8. Process according to any of the preceding claims, **characterized in that** the oxygenate partial pressure inside the catalyst stage is between 0.1 and 0.5 bar(a).

9. Process according to Claim 1, **characterized in that** the oxygenates-containing reactant mixture has a DME content between 50% and 70% by weight, preferably between 55% and 60% by weight.

10. Process according to Claim 1, **characterized in that** the absolute pressure of the oxygenates-containing reaction mixture before introduction into the OTO synthesis reactor is less than 7 bar(a), preferably less than 6 bar (a), and the temperature of the oxygenates-containing reaction mixture is set such that it is at least 5°C, preferably at least 10°C, above the dew point at this pressure.

11. Process according to Claim 1, **characterized in that** the absolute , pressure of the oxygenates-containing reaction mixture before introduction into the OTO synthesis reactor is less than 7 bar(a), preferably less than 6 bar(a), and the temperature of the oxygenates-containing reaction mixture is at least 140°C, preferably at least 150°C.

12. Plant for producing an olefins-containing hydrocarbon product comprising ethylene and propylene by conversion of an oxygenates-containing reactant mixture, which is divided into a plurality of reactant mixture substreams, in a multi-stage oxygenate-to-olefin (OTO) synthesis reactor comprising the following constituents:
(a) a multistage OTO synthesis reactor having a plurality of serially connected reaction sections in fluid connection with one another comprising a first reaction section and at least one subsequent reaction section which each contain catalyst zones comprising solid catalysts that are active and selective for OTO synthesis, wherein upstream of each catalyst zone a feeding apparatus for a reactant mixture substream is arranged and wherein the last reaction section in the direction of flow is in fluid connection with a conduit for discharging an OTO synthesis reactor product,
(b) means for introducing a reactant mixture substream into each reaction section via the respective feeding apparatus, means for introducing at least one steam stream into at least one reaction section, means for introducing at least one recycle stream into at least one reaction section,
(c) means for adjusting oxygenate conversion conditions, means for discharging the OTO synthesis reactor product,
(d) a multi-stage workup apparatus operating by means of thermal separation processes and suitable for separating the OTO synthesis reactor product into a plurality of hydrocarbons-containing hydrocarbon product fractions, means for introducing the OTO synthesis reactor product into the workup apparatus,
(e) means for discharging an olefins-containing, in particular ethylene- and/or propylene-containing, hydrocarbon product from the workup apparatus,
(f) means for recycling at least a portion of one or more hydrocarbon product fractions obtained in the workup apparatus to the OTO synthesis reactor as a recycle stream or recycle streams and means for introducing the recycle stream(s) into at least one reaction section,
**characterized in that** all means recited under (b) are configured such that all reactant mixture substreams, steam streams and recycle streams are introduceable into the OTO synthesis reactor in gaseous/vaporous form and **in that** said plant further comprises an etherification reactor arranged upstream of the OTO synthesis reactor which is configured such that by catalytic dehydration of methanol in the gas phase a gaseous, oxygenates-containing reactant mixture that can be sent to the OTO synthesis reactor without an additional separation step is obtainable.

13. Plant according to Claim 12, **characterized in that** all reaction sections are provided with means for introducing reactant mixture substreams on the one hand and with means for introducing steam streams and/or recycle streams on the other hand.

14. Plant according to Claim 12 or 13, **characterized in that** the workup apparatus comprises a plurality of separation stages in which different hydrocarbon fractions are obtained and further comprises at least two recycle conduits which recycle from different separation stages to the OTO synthesis reactor and which are connected to different means for introducing recycle streams into the reaction sections.

15. Plant according to Claim 14, **characterized in that** a first separation stage is connected to the first reaction section via a first recycle conduit and **in that** a second separation stage is connected to at least one subsequent reaction section via a second recycle conduit.

## Revendications

1. Procédé de fabrication de produits hydrocarbonés contenant des oléfines, comprenant de l'éthylène et du propylène, par réaction d'un mélange de réactifs contenant des oxygénats, qui est subdivisé en plusieurs courants partiels du mélange de réactifs, dans un réacteur de synthèse oxygénat-oléfines (OTO) à plusieurs étages, comprenant les étapes suivantes :
(a) fourniture! d'un réacteur de synthèse OTO à plusieurs étages, comportant plusieurs sections réactionnelles! montées les unes après les autres, en liaison fluidique les unes avec les autres, comprenant une première section réactionnelle et au moins une section réactionnelle suivante, dont chacune contient des zones de catalyseur comportant des catalyseurs solides, actifs pour la synthèse OTO et sélectifs, un dispositif d'addition d'un courant partiel du mélange de réactifs étant disposé en amont de chaque zone de catalyseur, et la dernière section réactionnelle aval étant en liaison fluidique avec une conduite d'évacuation d'un produit du réacteur de synthèse OTO,
(b) introduction d'un courant partiel du mélange de réactifs dans chaque section réactionnelle par chaque dispositif d'addition, l'au moins une section réactionnelle suivante recevant en outre le courant de produit de la section réactionnelle immédiatement en amont, introduction d'au moins un courant de vapeur d'eau dans au moins une section réactionnelle, introduction d'au moins un courant de retour dans au moins une section réactionnelle,
(c) réaction au moins partielle des oxygénats amenés dans les zones de catalyseur dans les conditions d'une réaction des oxygénats pour obtenir des oléfines et d'autres hydrocarbures, évacuation du produit du réacteur de synthèse OTO,
(d) séparation du produit du réacteur de synthèse OTO dans un dispositif de traitement à plusieurs étages, travaillant par séparation thermique, pour obtenir plusieurs fractions de produits hydrocarbonés contenant des hydrocarbures,
(e) évacuation, à partir du dispositif de traitement, d'un produit hydrocarboné contenant des oléfines, en particulier de l'éthylène et/ou du propylène,
(f) retour d'au moins une partie d'une ou plusieurs fractions de produits hydrocarbonés en tant que courant de retour ou courants de retour vers le réacteur de synthèse OTO et introduction du ou des courants de retour dans au moins une section réactionnelle,
**caractérisé en ce que** la totalité des courants partiels du mélange de réactifs, des courants de vapeur d'eau et des courants de retour sont introduits exclusivement sous forme gazeuse ou vapeur dans le réacteur de synthèse OTO, et **en ce que** le mélange de réactifs contenant des oxygénats contient du diméthyléther (DME) et est produit dans un réacteur d'éthérification par déshydratation catalytique du méthanol en phase gazeuse, avec obtention d'un mélange gazeux de produits du réacteur d'éthérification, qui comprend du DME, de la vapeur d'eau et de la vapeur de méthanol, le mélange gazeux de produits du réacteur d'éthérification étant, sans étape supplémentaire de séparation, envoyé sous forme d'un mélange de réactifs dans le réacteur de synthèse OTO. ;

2. Procédé selon la revendication 1, **caractérisé en ce que** toutes les sections réactionnelles reçoivent des courants partiels du mélange de réactifs d'une part et d'autre part des courants de vapeur d'eau et/ou des courants de retour.

3. Procédé selon la revendication 1 ou 2, **caractérisé en ce qu'**au moins deux fractions de produits hydrocarbonés sont renvoyées sous forme de courants de retour au réacteur de synthèse OTO et y sont introduites.

4. Procédé selon la revendication 3, **caractérisé en ce qu'**une fraction de produits hydrocarbonés contenant essentiellement des hydrocarbures en C₂ à C₈ est introduite sous forme d'un courant de retour dans la première section réactionnelle.

5. Procédé selon la revendication 3 ou 4, **caractérisé en ce qu'**exclusivement une fraction de produits hydrocarbonés contenant essentiellement des hydrocarbures en C₂ à C₄ est introduite sous forme d'un courant de retour dans au moins une section réactionnelle suivante.

6. Procédé selon l'une des revendications précédentes, **caractérisé en ce que** la perte de charge, dans un dispositif d'addition d'un courant partiel de mélange de réactifs est inférieure à 5 bar(a), de préférence inférieure à 3 bar(a).

7. Procédé selon l'une des revendications précédentes, **caractérisé en ce que** le débit massique du courant de retour et/ou le débit massique de la vapeur d'eau sont pilotés et régulés séparément pour au moins deux sections réactionnelles.

8. Procédé selon l'une des revendications précédentes, **caractérisé en ce que** la pression partielle de l'oxygénat à l'intérieur de l'étage de catalyseur est comprise entre 0,1 et 0,5 bar(a).

9. Procédé selon la revendication 1, **caractérisé en ce que** le mélange de réactifs contenant des oxygénats présente une teneur en DME entre 50 et 70 % en poids, de préférence entre 55 et 60 % en poids.

10. Procédé selon la revendication 1, **caractérisé en ce que** la pression absolue du mélange de réactifs contenant des oxygénats est, avant introduction dans le réacteur de synthèse OTO, inférieure à 7 bar(a), de préférence inférieure à 6 bar(a), et la température du mélange de réactifs contenant des oxygénats est ajustée de telle sorte qu'elle soit d'au moins 5 °C, de préférence d'au moins 10 °C, supérieure au point de rosée sous cette pression.

11. Procédé selon la revendication 1, **caractérisé en ce que** la pression absolue du mélange de réactifs contenant des oxygénats est, avant introduction dans le réacteur de synthèse OTO, inférieure à 7 bar(a), de préférence inférieure à 6 bar(a), et la température du mélange de réactifs contenant des oxygénats est d'au moins 140 °C, de préférence d'au moins 150 °C.

12. Installation de fabrication d'un produit hydrocarboné contenant des oléfines, comprenant de l'éthylène et du propylène, par réaction d'un mélange de réactifs contenant des oxygénats, qui est subdivisé en plusieurs courants partiels du mélange de réactifs, dans un réacteur de synthèse oxygénat-oléfine (OTO) à plusieurs étages, comprenant les constituants suivants :
(a) un réacteur de synthèse OTO à plusieurs étages, comportant plusieurs sections réactionnelles montées les unes derrière les autres, en liaison fluidique les unes avec les autres, comprenant une première section réactionnelle et au moins une section réactionnelle suivante, dontchacune contient des zones de catalyseur contenant des catalyseurs solides, actifs pour la synthèse OTO et sélectifs, un dispositif d'addition pour un courant partiel du mélange de réactifs étant disposé en amont de chaque zone de catalyseur, et la dernière section réactionnelle aval étant en liaison fluidique avec une conduite pour évacuation d'un produit du réacteur de synthèse OTO,
(b) des moyens pour introduire un courant partiel du mélange de réactifs dans chaque section réactionnelle par le dispositif d'addition correspondant, des moyens pour introduire au moins un courant de vapeur d'eau dans au moins une section réactionnelle, des moyens pour introduire au moins un courant de retour dans au moins une section réactionnelle,
(c) des moyens pour ajuster les conditions de réaction de l'oxygénat, des moyens pour évacuer le produit du réacteur de synthèse OTO,
(d) un dispositif à plusieurs étages travaillant par séparation thermique, convenant à la séparation du produit du réacteur de synthèse OTO en plusieurs fractions de produits hydrocarbonés contenant des hydrocarbures, des moyens pour introduire le produit du réacteur de : synthèse OTO dans le dispositif de traitement,
(e) des moyens pour évacuer un produit hydrocarboné contenant des oléfines, en particulier de l'éthylène et/ou du propylène, à partir du dispositif de traitement,
(f) des moyens pour retourner au moins une partie d'une ou plusieurs des fractions de produits hydrocarbonés obtenues dans : le dispositif de travail, en tant que courant de retour ou courants de retour vers le réacteur de synthèse OTO et des moyens pour introduire dans au moins une section réactionnelle le ou les courants de retour,
**caractérisée en ce que** tous les moyens mentionnés en (b) sont conçus de telle sorte que la totalité des courants partiels de mélange de réactifs, des courants de vapeur d'eau et des courants de retour peuvent être introduits sous forme gazeuse ou vapeur dans le réacteur de synthèse OTO, et **en ce qu'**en outre il est prévu un réacteur d'éthérification monté en aval du réacteur de synthèse OTO, qui est conçu de de telle sorte qu'un mélange gazeux de réactifs, contenant des oxygénats, peut être produit par déshydratation catalytique du méthanol en phase gazeuse, mélange qui, sans une étape de séparation supplémentaire, peut être envoyé au réacteur de synthèse OTO.

13. Installation selon la revendication 12, **caractérisée en ce que** toutes les sections réactionnelles sont équipées de moyens pour introduire des courants partiels de mélange de réactifs d'une part, et d'autre part des moyens pour introduire des courants de vapeur d'eau et/ou des courants de retour.

14. Installation selon la revendication 12 ou 13, **caractérisée en ce que** le dispositif de traitement comprend plusieurs étapes de séparation, dans lesquelles on obtient différentes fractions hydrocarbonées, et comprenant en outre au moins deux conduites de retour, qui renvoient des différents étages de séparation au réacteur de synthèse OTO, et qui sont reliées à différents moyens pour introduire des courants de retour dans les sections réactionnelles.

15. Installation selon la revendication 14, **caractérisée en ce qu'**un premier étage de séparation est, par l'intermédiaire d'une première conduite de retour, relié à la première section réactionnelle, et **en ce qu'**un deuxième étage de séparation est, par l'intermédiaire d'une deuxième conduite de retour, relié à au moins une section réactionnelle suivante.
